# EUROPEAN PATENT SPECIFICATION

(11) **EP 1 230 344 B1**
(45) Date of publication and mention of the grant of the patent: **02.11.2011**
(21) Application number: 00980390.9
(22) Date of filing: 14.11.2000
(51) Int. Cl.: C12N 15/82, C07K 14/415, A01H 5/00

(54) **Plant biochemistry-related genes**
Gene im Zusammenhang mit der Biochemie von Pflanzen
Gènes lies à la biochimie des végétaux

(30) Priority: 17.11.1999 US 166228 P; 17.04.2000 US 197899 P; 22.08.2000 US 227439 P
(43) Date of publication of application: 14.08.2002
(73) Proprietor: Mendel Biotechnology, Inc., Hayward, CA 94545 (US)
(72) Inventor: CREELMAN, Robert, Castro Valley, CA 94546 (US); YU, Guo-Liang, Berkeley, CA 94705 (US); ADAM, Luc, Hayward, CA 94545 (US); RIECHMANN, Jose Luis, Pasadena, CA 91101 (US); HEARD, Jacqueline, San Mateo, CA 94402 (US); SAMAHA, Raymond, Capitola, CA 95010 (US); PILGRIM, Marsha, Fremont, CA 94536 (US); PINEDA, Omaira, Vero Beach, FL 32960 (US); JIANG, Cai-Zhong, Fremont, CA 94555 (US)
(74) Representative: Brasnett, Adrian Hugh
(86) International application number: PCT/US2000/031344
(87) International publication number: WO 2001/036597

(56) References cited:
- EP-A- 0 776 973
- EP-A- 1 033 405
- WO-A-99/41974
- WO-A-99/55840
- WO-A1-98/48007
- WANG ZHI-YONG ET AL: "A Myb-related transcription factor is involved in the phytochrome regulation of an Arabidopsis Lhcb gene" PLANT CELL, AMERICAN SOCIETY OF PLANT PHYSIOLOGISTS, ROCKVILLE, MD, US, vol. 9, no. 4, 1997, pages 491-507, XP002296200 ISSN: 1040-4651
- WANG Z-Y ET AL: "CONSTITUTIVE EXPRESSION OF THE CIRCADIAN CLOCK ASSOCIATED 1 (CCA1) GENE DISRUPTS CIRCADIAN RHYTHMS AND SUPPRESSES ITS OWN EXPRESSION" CELL, CELL PRESS, CAMBRIDGE, NA, US, vol. 93, no. 7, 1998, pages 1207-1217, XP000929262 ISSN: 0092-8674
- MARTIN C ET AL: "MYB transcription factors in plants" TRENDS IN GENETICS, ELSEVIER SCIENCE PUBLISHERS B.V. AMSTERDAM, NL, vol. 13, no. 2, 1 February 1997 (1997-02-01), pages 67-73, XP004034152 ISSN: 0168-9525
- JIN HAILING ET AL: "Multifunctionality and diversity within the plant MYB-gene family" PLANT MOLECULAR BIOLOGY, vol. 41, no. 5, November 1999 (1999-11), pages 577-585, XP002302993 ISSN: 0167-4412
- BRAUN EDWARD L ET AL: "Newly discovered plant c-myb-like genes rewrite the evolution of the plant myb gene family" PLANT PHYSIOLOGY (ROCKVILLE), vol. 121, no. 1, September 1999 (1999-09), pages 21-24, XP002302994 ISSN: 0032-0889
- SHEWMAKER C K ET AL: "Seed-specific overexpression of phytoene synthase: increase in carotenoids and other metabolic effects" PLANT JOURNAL, BLACKWELL SCIENTIFIC PUBLICATIONS, OXFORD, GB, vol. 20, no. 4, 1999, pages 401-412, XP002969263 ISSN: 0960-7412
- DATABASE EMBL [Online] 21 May 1999 (1999-05-21), "Arabidopsis thaliana chromosome 1 BAC F9L1 sequence, complete sequence." XP002312432 retrieved from EBI accession no. EM_PRO:AC007591 Database accession no. AC007591
- DATABASE EMBL [Online] 9 January 1998 (1998-01-09), "33655 CD4-6 Arabidopsis thaliana cDNA clone K2E2T7 3', mRNA sequence." XP002312433 retrieved from EBI accession no. EM_PRO:AA728476 Database accession no. AA728476
- DATABASE EMBL [Online] 13 October 1997 (1997-10-13), "T26B22TRD TAMU Arabidopsis thaliana genomic clone T26B22, genomic survey sequence." XP002312435 retrieved from EBI accession no. EM_PRO:B30104 Database accession no. B30104
- SOLANO R ET AL: "NUCLEAR EVENTS IN ETHYLENE SIGNALING: A TRANSCRIPTIONAL CASCADE MEDIATED BY ETHYLENE-INSENSITIVE3 AND ETHYLENE-RESPONSE-FACTOR1" GENES AND DEVELOPMENT, COLD SPRING HARBOR LABORATORY PRESS, NEW YORK, US, vol. 12, 1 December 1998 (1998-12-01), pages 3703-3714, XP000971358 ISSN: 0890-9369
- MOOSE S P ET AL: "GLOSSY15, AN APETALA2-LIKE GENE FROM MAIZE THAT REGULATES LEAF EPIDERMAL CELL IDENTITY" GENES AND DEVELOPMENT, COLD SPRING HARBOR, NY, US, vol. 10, 1996, pages 3018-3027, XP000989661 ISSN: 0890-9369
- OKAMURO J ET AL: "The AP2 domain of APETALA2 defines a large new family of DNA binding proteins in Arabidopsis" PROCEEDINGS OF THE NATIONAL ACADEMY OF SCIENCES OF USA, NATIONAL ACADEMY OF SCIENCE. WASHINGTON, US, vol. 94, no. 13, June 1997 (1997-06), pages 7076-7081, XP002108957 ISSN: 0027-8424
- XIA YIJI ET AL: "Developmental and hormonal regulation of the Arabidopsis CER2 gene that codes for a nuclear-localized protein required for the normal accumulation of cuticular waxes" PLANT PHYSIOLOGY (ROCKVILLE), vol. 115, no. 3, November 1997 (1997-11), pages 925-937, XP002312431 ISSN: 0032-0889
- BROUN P ET AL: "WIN1, a transcriptional activator of epidermal wax accumulation in Arabidopsis" PROCEEDINGS OF THE NATIONAL ACADEMY OF SCIENCES OF USA, NATIONAL ACADEMY OF SCIENCE. WASHINGTON, US, vol. 101, no. 13, 30 March 2004 (2004-03-30), pages 4706-4711, XP002300982 ISSN: 0027-8424
- AHARONI A ET AL: "The SHINE clade of AP2 domain transcription factors activates wax biosynthesis, alters cuticle properties, and confers drought tolerance when overexpressed in Arabidopsis" PLANT CELL, AMERICAN SOCIETY OF PLANT PHYSIOLOGISTS, ROCKVILLE, MD, US, vol. 16, no. 9, September 2004 (2004-09), pages 2463-2480, XP002300985 ISSN: 1040-4651
- DATABASE GENEMBL [Online] 14 July 1997 WANG ET AL.: 'Arabidopsis thaliana DNA-binding protein CCAL(CCAL) mRNA, complete cds', XP002937902 Database accession no. U28422
- DATABASE GENESEQ [Online] THE REGENTS OF THE UNIVERSITY OF CALIFORNIA 15 February 1999 XP002937903 Database accession no. V65382
- DATABASE PIR [Online] 24 March 1999 ROUNSLEY ET AL.: 'DNA-binding protein CCAL - Arabidopsis thaliana', XP002937904 Database accession no. T02684
- DATABASE GENESEQ [Online] THE REGENTS OF THE UNIVERSITY OF CALIFORNIA 15 February 1999 XP002937905 Database accession no. W79280
- RIECHMANN ET AL.: 'A genomic perspective on plant transcription factors' CURRENT OPINION IN PLANT BIOLOGY vol. 3, no. 5, October 2000, pages 423 - 434, XP002937906
- RIECHMANN ET AL.: 'The AP2/EREBP family of plant transcription factors' BIOLOGICAL CHEMISTRY vol. 379, no. 6, June 1998, pages 633 - 646, XP002937907
- RIECHMANN ET AL.: 'MADS domain proteins in plant development' BIOLOGICAL CHEMISTRY vol. 378, no. 10, October 1997, pages 1079 - 1101, XP002937908
- HEARD ET AL.: 'Evolutionary diversity of symbiotically induced nodule MADS box genes: Characterization of nmhC5. A member of a novel subfamily' MOLECULAR PLANT-MICROBE INTERACTIONS: MPMI vol. 10, no. 5, July 1997, pages 665 - 676, XP002937909
- HEARD ET AL.: 'Symbiotic induction of a MADS-box gene during development of alfafa root nodules' PROC. NATL. ACAD. SCI. USA vol. 92, no. 12, 06 June 1995, pages 5273 - 5277, XP002937910

## Description

### FIELD OF THE INVENTION

This invention relates to the field of plant biology. More particularly, the present invention pertains to compositions and methods for phenotypically modifying a plant.

### BACKGROUND OF THE INVENTION

Transcription factors can modulate gene expression, either increasing or decreasing (inducing or repressing) the rate of transcription. This modulation results in differential levels of gene expression at various developmental stages, in different tissues and cell types, and in response to different exogenous (e.g., environmental) and endogenous stimuli throughout the life cycle of the organism.

Because transcription factors are key controlling elements of biological pathways, altering the expression levels of one or more transcription factors can change entire biological pathways in an organism. For example, manipulation of the levels of selected transcription factors may result in increased expression of economically useful proteins or metabolic chemicals in plants or to improve other agriculturally relevant characteristics. Conversely, blocked or reduced expression of a transcription factor may reduce biosynthesis of unwanted compounds or remove an undesirable trait. Therefore, manipulating transcription factor levels in a plant offers tremendous potential in agricultural biotechnology for modifying a plant's traits.

The present invention provides the use of transcription factors for modifying a plant's phenotype in desirable ways, such as modifying a plant's biochemical traits.

### SUMMARY OF THE INVENTION

In a first aspect, the invention relates to the use of a nucleotide sequence which encodes a transcription factor polypeptide comprising a sequence having at least 50% sequence identity to SEQ ID NO:28 for producing a transgenic plant having the trait of increased wax content in leaves by over-expression of said polypeptide.

In a second aspect, the invention provides a method for producing transgenic plant having the trait of increased wax content in leaves, said method comprising:
introducing into a plant, plant cell, plant explant, or plant tissue an expression vector comprising a nucleotide sequence which encodes a transcription factor polypeptide comprising a sequence having at least 50% sequence identity to the SEQ ID NO:28;
producing a transgenic plant having said expression vector encoding said transcription factor polypeptide; and
selecting a plant having said trait of increased wax content in leaves caused by over-expression of said polypeptide.

In a third aspect, the invention provides a transgenic plant produced by the method of the second aspect of the invention, or progeny thereof, wherein said plant comprises an expression vector comprising a nucleotide sequence which encodes a polypeptide having at least 50% sequence identity to SEQ ID NO:28 and said plant has the trait of increased wax content in leaves caused by ectopic over-expression of said transcription factor polypeptide.

In a fourth aspect, the invention provides a seed of the transgenic plant, said seed comprising an expression vector comprising a nucleotide sequence which encodes a polypeptide having at least 50% sequence identity to SEQ ID NO:28.

In yet a further aspect, described below is a composition produced by incubating a polynucleotide described herein with a nuclease, a restriction enzyme, a polymerase; a polymerase and a primer; a cloning vector, or with a cell.

Furthermore, the invention relates to a method for producing a plant having a modified biochemical trait. The method comprises altering the expression of an isolated or recombinant polynucleotide described herein or altering the expression or activity of a polypeptide of the invention in a plant to produce a modified plant, and selecting the modified plant for a modified biochemical trait.

In another aspect, described herein is a method of identifying a factor that is modulated by or interacts with a polypeptide encoded by a polynucleotide described herein. The method comprises expressing a polypeptide encoded by the polynucleotide in a plant; and identifying at least one factor that is modulated by or interacts with the polypeptide. In one embodiment the method for identifying modulating or interacting factors is by detecting binding by the polypeptide to a promoter sequence, or by detecting interactions between an additional protein and the polypeptide in a yeast two hybrid system, or by detecting expression of a factor by hybridization to a microarray, subtractive hybridization or differential display.

In yet another aspect, described herein is a method of identifying a molecule that modulates activity or expression of a polynucleotide or polypeptide of interest. The method comprises placing the molecule in contact with a plant comprising the polynucleotide or polypeptide encoded by the polynucleotide described herein and monitoring one or more of the expression level of the polynucleotide in the plant, the expression level of the polypeptide in the plant, and modulation of an activity of the polypeptide in the plant.

In yet another aspect, described herein is an integrated system, computer or computer readable medium comprising one or more character strings corresponding to a polynucleotide described herein, or to a polypeptide encoded by the polynucleotide. The integrated system, computer or computer readable medium may comprise a link between one or more sequence strings to a modified plant biochemical trait.

In yet another aspect, described herein is a method for identifying a sequence similar or homologous to one or more polynucleotides described herein, or one or more polypeptides encoded by the polynucleotides. The method comprises providing a sequence database; and, querying the sequence database with one or more target sequences corresponding to the one or more polynucleotides or to the one or more polypeptides to identify one or more sequence members of the database that display sequence similarity or homology to one or more of the one or more target sequences.

The method may further comprise of linking the one or more of the polynucleotides described herein, or encoded polypeptides, to a modified plant biochemical phenotype.

### TABLES

The polynucleotide and polypeptide sequence to which the invention relates is set out in the table below. The table includes from left to right for each sequence: the SEQ ID No., the internal code reference number (GID), whether the sequence is a polynucleotide or polypeptide sequence, and identification of any conserved domains for the polypeptide sequence:

| SEQ ID No. | GID | cDNA or protein | conserved domain |
|---|---|---|---|
| 27 | G975 | cDNA | |
| 28 | G975 | protein | 4-71 |

The table below sets out a sequence that is homologous to the G975 of the Sequence Listing and, that is derived from *Arabidopsis thaliana.* The table includes from left to right: the SEQ ID No. the internal code reference number (GID), identification of the homologous sequence, whether the sequence is a polynucleotide or polypeptide sequence, and identification of any conserved domains for the polypeptide sequence:

| SEQ ID No. | GID | homolog | cDNA or protein | conserved domain |
|---|---|---|---|---|
| 43 | G1387 | G975 | cDNA | |
| 44 | G1387 | G975 | protein | 4-71 |

### BRIEF DESCRIPTION OF THE DRAWINGS

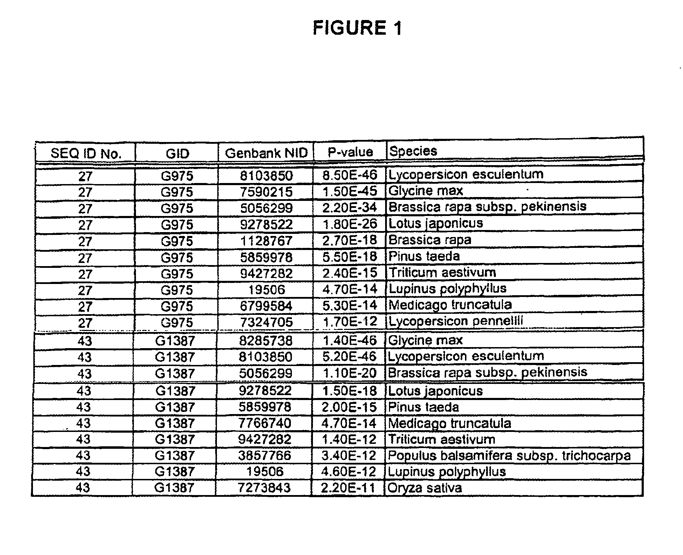

Figure 1 provides a table of exemplary sequences that are homologous to the sequences provided in the Tables above and that are derived from plants other than *Arabidopsis thaliana.* The table includes from left to right: the SEQ ID No., the internal code reference number (GID), the unique GenBank sequence ID No. (NID), the probability that the comparison was generated by chance (P-value), and the species from which the homologous gene was identified.

### DETAILED DESCRIPTION

Plant transcription factors derived, e.g., from *Arabidopsis thaliana* can belong, e.g., to one or more of the following transcription factor families: the AP2 (APETALA2) domain transcription factor family (Riechmann and Meyerowitz (1998) J. Biol. Chern. 379:633-646); the MYB transcription factor family (Martin and Paz-Ares (1997) Trends Genet. 13:67-73); the MADS domain transcription factor family (Riechmann and Meyerowitz (1997) J.Biol.Chem. 378:10791101); the WRKY protein family (Ishiguro and Nakamura (1994) Mol.Gen.Genet. 244: 563571); the ankyrin-repeat protein family (Zhang et al.(1992) Plant Cell 4: 1575-1588); the miscellaneous protein (MISC) family (Kim et al. (1997) Plant J. 11:1237-1251); the zinc finger protein (Z) family (Klug and Schwabe (1995) FASEB J. 9: 597-604); the homeobox (HB) protein family (Duboule (1994) Guidebook to the Homeobox Genes. Oxford University Press); the CAAT-element binding proteins (Forsburg and Guarente (1989) Genes Dev. 3:1166-1178); the squamosa promoter binding proteins (SPB) (Klein et al. (1996) Mol. Gen. Genet. 1996 250:7-16); the NAM protein family; the IAA/AUX proteins (Rouse et al. (1998) Science 279:1371-1373); the HLH/MYC protein family (Littlewood et al. (1994) Prot. Profile 1:639-709); the DNA-binding protein (DBP) family (Tucker et al. (1994) EMBO J. 13:2994-3002); the bZIP family of transcription factors (Foster et al. (1994) FASEB J. 8:192-200); the BPF-1 protein (Box P-binding factor) family (da Costa e Silva et al. (1993) Plant J. 4:125-135); and the golden protein (GLD) family (Hall et al. (1998) Plant Cell 10:925-936).

In addition to methods for modifying a plant phenotype by employing one or more polynucleotides and polypeptides described herein, the polynucleotides and polypeptides have a variety of additional uses. These uses include their use in the recombinant production (i.e, expression) of proteins; as regulators of plant gene expression, as diagnostic probes for the presence of complementary or partially complementary nucleic acids (including for detection of natural coding nucleic acids); as substrates for further reactions, e.g., mutation reactions, PCR reactions, or the like, of as substrates for cloning e.g., including digestion or ligation reactions, and for identifying exogenous or endogenous modulators of the transcription factors.

### DEFINITIONS

A "polynucleotide" is a nucleic acid sequence comprising a plurality of polymerized nucleotide residues, e.g., at least about 15 consecutive polymerized nucleotide residues, optionally at least about 30 consecutive nucleotides, at least about 50 consecutive nucleotides. In many instances, a polynucleotide comprises a nucleotide sequence encoding a polypeptide (or protein) or a domain or fragment thereof. Additionally, the polynucleotide may comprise a promoter, an intron, an enhancer region, a polyadenylation site, a translation initiation site, 5' or 3' untranslated regions, a reporter gene, a selectable marker, or the like. The polynucleotide can be single stranded or double stranded DNA or RNA. The polynucleotide optionally comprises modified bases or a modified backbone. The polynucleotide can be, e.g., genomic DNA or RNA, a transcript (such as an mRNA), a cDNA, a PCR product, a cloned DNA, a synthetic DNA or RNA, or the like. The polynucleotide can comprise a sequence in either sense or antisense orientations.

A "recombinant polynucleotide" is a polynucleotide that is not in its native state, e.g., the polynucleotide comprises a nucleotide sequence not found in nature, or the polynucleotide is in a context other than that in which it is naturally found, e.g., separated from nucleotide sequences with which it typically is in proximity in nature, or adjacent (or contiguous with) nucleotide sequences with which it typically is not in proximity. For example, the sequence at issue can be cloned into a vector, or otherwise recombined with one or more additional nucleic acid.

An "isolated polynucleotide" is a polynucleotide whether naturally occurring or recombinant, that is present outside the cell in which it is typically found in nature, whether purified or not. Optionally, an isolated polynucleotide is subject to one or more enrichment or purification procedures, e.g., cell lysis, extraction, centrifugation, precipitation, or the like.

A "recombinant polypeptide" is a polypeptide produced by translation of a recombinant polynucleotide. An "isolated polypeptide," whether a naturally occurring or a recombinant polypeptide, is more enriched in (or out of) a cell than the polypeptide in its natural state in a wild type cell, e.g., more than about 5% enriched, more than about 10% enriched, or more than about 20%, or more than about 50%, or more, enriched, i.e., alternatively denoted: 105%, 110%, 120%, 150% or more, enriched relative to wild type standardized at 100%. Such an enrichment is not the result of a natural response of a wild type plant. Alternatively, or additionally, the isolated polypeptide is separated from other cellular components with which it is typically associated, e.g., by any of the various protein purification methods herein.

The term "transgenic plant" refers to a plant that contains genetic material, not found in a wild type plant of the same species, variety or cultivar. The genetic material may include a transgene, an insertional mutagenesis event (such as by transposon or T-DNA insertional mutagenesis), an activation tagging sequence, a mutated sequence, a homologous recombination event or a sequence modified by chimeraplasty. Typically, the foreign genetic material has been introduced into the plant by human manipulation.

A transgenic plant may contain an expression vector or cassette. The expression cassette typically comprises a polypeptide-encoding sequence operably linked (i.e., under regulatory control of) to appropriate inducible or constitutive regulatory sequences that allow for the expression of polypeptide. The expression cassette can be introduced into a plant by transformation or by breeding after transformation of a parent plant. A plant refers to a whole plant as well as to a plant part, such as seed, fruit, leaf, or root, plant tissue, plant cells or any other plant material, e.g., a plant explant, as well as to progeny thereof, and to *in vitro* systems that mimic biochemical or cellular components or processes in a cell.

The phrase "ectopically expression or altered expression" in reference to a polynucleotide indicates that the pattern of expression in, e.g., a transgenic plant or plant tissue, is different from the expression pattern in a wild type plant or a reference plant of the same species. For example, the polynucleotide or polypeptide is expressed in a cell or tissue type other than a cell or tissue type in which the sequence is expressed in the wild type plant, or by expression at a time other than at the time the sequence is expressed in the wild type plant, or by a response to different inducible agents, such as hormones or environmental signals, or at different expression levels (either higher or lower) compared with those found in a wild type plant. The term also refers to altered expression patterns that are produced by lowering the levels of expression to below the detection level or completely abolishing expression. The resulting expression pattern can be transient or stable, constitutive or inducible. In reference to a polypeptide, the term "ectopic expression or altered expression" further may relate to altered activity levels resulting from the interactions of the polypeptides with exogenous or endogenous modulators or from interactions with factors or as a result of the chemical modification of the polypeptides.

The term "fragment" or "domain," with respect to a polypeptide, refers to a subsequence of the polypeptide. In some cases, the fragment or domain, is a subsequence of the polypeptide which performs at least one biological function of the intact polypeptide in substantially the same manner, or to a similar extent, as does the intact polypeptide. For example, a polypeptide fragment can comprise a recognizable structural motif or functional domain such as a DNA binding domain that binds to a DNA promoter region, an activation domain or a domain for protein-protein interactions. Fragments can vary in size from as few as 6 amino acids to the full length of the intact polypeptide, but are preferably at least about 30 amino acids in length and more preferably at least about 60 amino acids in length. In reference to a nucleotide sequence, "a fragment" refers to any subsequence of a polynucleotide, typically, of at least consecutive about 15 nucleotides, preferably at least about 30 nucleotides, more preferably at least about 50, of any of the sequences provided herein.

The term "trait" refers to a physiological, morphological, biochemical or physical characteristic of a plant or particular plant material or cell. In some instances, this characteristic is visible to the human eye, such as seed or plant size, or can be measured by available biochemical techniques, such as the protein, starch or oil content of seed or leaves or by the observation of the expression level of genes, e.g., by employing Northern analysis, RT-PCR, microarray gene expression assays or reporter gene expression systems, or by agricultural observations such as stress tolerance, yield or pathogen tolerance.

"Trait modification" refers to a detectable difference in a characteristic in a plant ectopically expressing a polynucleotide or polypeptide of the present invention relative to a plant not doing so, such as a wild type plant. In some cases, the trait modification can be evaluated quantitatively. For example, the trait modification can entail at least about a 2% increase or decrease in an observed trait (difference), at least a 5% difference, at least about a 10% difference, at least about a 20% difference, at least about a 30%, at least about a 50%, at least about a 70%, or at least about a 100%, or an even greater difference. It is known that there can be a natural variation in the modified trait. Therefore, the trait modification observed entails a change of the normal distribution of the trait in the plants compared with the distribution observed in wild type plant.

Trait modifications of particular interest include those to seed (such as embryo or endosperm), fruit; root, flower, leaf, stem, shoot, seedling or the like, including: enhanced tolerance to environmental conditions including freezing, chilling, heat, drought, water saturation, radiation and ozone; improved tolerance to microbial, fungal or viral diseases; improved tolerance to pest infestations, including nematodes, mollicutes, parasitic higher plants or the like; decreased herbicide sensitivity; improved tolerance of heavy metals or enhanced ability to take up heavy metals; improved growth under poor photoconditions (e.g., low light and/or short day length), or changes in expression levels of genes of interest. Other phenotype that can be modified relate to the production of plant metabolites, such as variations in the production of taxol, tocopherol, tocotrienol, sterols, phytosterols, vitamins, wax monomers, anti-oxidants, amino acids, lignins, cellulose, tannins, prenyllipids (such as chlorophylls and carotenoids), glucosinolates, and terpenoids, enhanced or compositionally altered protein or oil production (especially in seeds), or modified sugar (insoluble or soluble) and/or starch composition Physical plant characteristics that can be modified include cell development (such as the number of trichomes), fruit and seed size and number, yields of plant parts such as stems, leaves and roots, the stability of the seeds during storage, characteristics of the seed pod (e.g., susceptibility to shattering), root hair length and quantity, internode distances, or the quality of seed coat. Plant growth characteristics that can be modified include growth rate, germination rate of seeds, vigor of plants and seedlings, leaf and flower senescence, male sterility, apomixis, flowering time, flower abscission, rate of nitrogen uptake, biomass or transpiration characteristics, as well as plant architecture characteristics such as apical dominance, branching patterns, number of organs, organ identity, organ shape or size.

### POLYPEPTIDES AND POLYNUCLEOTIDES

Described herein are among other things, transcription factors (TFs), and transcription factor homologue polypeptides, and isolated or recombinant polynucleotides encoding the polypeptides. These polypeptides and polynucleotides may be employed to modify a plant's biochemical characteristic.

Exemplary polynucleotides encoding the polypeptides were identified in the *Arabidopsis thaliana* GenBank database using publicly available sequence analysis programs and parameters. Sequences initially identified were then further characterized to identify sequences comprising specified sequence strings corresponding to sequence motifs present in families of known transcription factors. Polynucleotide sequences meeting such criteria were confirmed as transcription factors.

Additional polynucleotides were identified by screening *Arabidopsis thaliana* and/or other plant cDNA libraries with probes corresponding to known transcription factors under low stringency hybridization conditions. Additional sequences, including full length coding sequences were subsequently recovered by the rapid amplification of cDNA ends (RACE) procedure, using a commercially available kit according to the manufacturer's instructions. Where necessary, multiple rounds of RACE are performed to isolate 5' and 3' ends. The full length cDNA was then recovered by a routine end-to-end polymerase chain reaction (PCR) using primers specific to the isolated 5' and 3' ends. Exemplary sequences are provided in the Sequence Listing.

The polynucleotides were ectopically expressed in overexpressor or knockout plants and changes in the biochemical characteristics of the plants were observed. Therefore, the polynucleotides and polypeptides can be employed to improve the biochemical characteristics of plants.

### Making polynucleotides

The polynucleotides include sequences that encode transcription factors and transcription factor homologue polypeptides and sequences complementary thereto, as well as unique fragments of coding sequence, or sequence complementary thereto. Such polynucleotides can be, e.g., DNA or RNA, e.g., mRNA, cRNA, synthetic RNA, genomic DNA. cDNA synthetic DNA, oligonucleotides, etc The polynucleotides are either double-stranded or single-stranded, and include either, or both sense (i.e., coding) sequences and antisense (i.e., non-coding, complementary) sequences. The polynucleotides include the coding sequence of a transcription factor, or transcription factor homologue polypeptide, in isolation, in combination with additional coding sequences (e.g., a purification tag, a localization signal, as a fusion-protein, as a pre-protein, or the like), in combination with non-coding sequences (e.g., introns or inteins, regulatory elements such as promoters, enhancers, terminators, and the like), and/or in a vector or host environment in which the polynucleotide encoding a transcription factor or transcription factor homologue polypeptide is an endogenous or exogenous gene.

A variety of methods exist for producing the polynucleotides described herein. Procedures for identifying and isolating DNA clones are well known to those of skill in the art, and are described in, e.g., Berger and Kimmel, Guide to Molecular Cloning Techniques. Methods in Enzymology volume 152 Academic Press, Inc., San Diego, CA ("Berger"); Sambrook et al., Molecular Cloning - A Laboratory Manual (2nd Ed.), Vol. 1-3, Cold Spring Harbor Laboratory, Cold Spring Harbor, New York, 1989 ("Sambrook") and Current Protocols in Molecular Biology, F.M. Ausubel et al., eds., Current Protocols, a joint venture between Greene Publishing Associates, Inc. and John Wiley & Sons, Inc.; (supplemented through 2000) ("Ausubel")

Alternatively, polynucleotides can be produced by a variety of in vitro amplification methods adapted to the present invention by appropriate selection of specific or degenerate primers. Examples of protocols sufficient to direct persons of skill through in vitro amplification methods, including the polymerase chain reaction (PCR) the ligase chain reaction (LCR), Qbeta-replicase amplification and other RNA polymerase mediated techniques (e.g., NASBA), e.g., for the production of the homologous nucleic acids of the invention are found in Berger, Sambrook, and Ausubel, as well as Mullis et al., (1987) PCR Protocols A Guide to Methods and Applications (Innis et al. eds) Academic Press Inc. San Diego, CA (1990) (Innis). Improved methods for cloning in vitro amplified nucleic acids are described in Wallace et al., U.S. Pat. No. 5,426,039. Improved methods for amplifying large nucleic acids by PCR are summarized in Cheng et al. (1994) Nature 369: 684-685 and the references cited therein, in which PCR amplicons of up to 40kb are generated. One of skill will appreciate that essentially any RNA can be converted into a double stranded DNA suitable for restriction digestion, PCR expansion and sequencing using reverse transcriptase and a polymerase. *See,* e.g., Ausubel, Sambrook and Berger, *all supra.*

Alternatively, polynucleotides and oligonucleotides can be assembled from fragments produced by solid-phase synthesis methods. Typically, fragments of up to approximately 100 bases are individually synthesized and then enzymatically or chemically ligated to produce a desired sequence, e.g., a polynucletotide encoding all or part of a transcription factor. For example, chemical synthesis using the phosphoramidite method is described, e.g., by Beaucage et al. (1981) Tetrahedron Letters 22:1859-69; and Matthes et al. (1984) EMBO J. 3:801-5. According to such methods, oligonucleotides are synthesized, purified, annealed to their complementary strand, ligated and then optionally cloned into suitable vectors. And if so desired, the polynucleotides and polypeptides can be custom ordered from any of a number of commercial suppliers.

### HOMOLOGOUS SEQUENCES

Sequences homologous, i.e., that share significant sequence identity or similarity, to those provided in the Sequence Listing derived from *Arabidopsis thaliana* or from other plants of choice are also described herein. Homologous sequences can be derived from any plant including monocots and dicots and in particular agriculturally important plant species, including but not limited to, crops such as soybean, wheat, corn, potato, cotton, rice, oilseed rape (including canola), sunflower, alfalfa, sugarcane and turf; or fruits and vegetables, such as banana, blackberry, blueberry, strawberry, and raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits (such as apple, peach, pear, cherry and plum) and vegetable brassicas (such as broccoli, cabbage, cauliflower, brussel sprouts and kohlrabi). Other crops, fruits and vegetables whose phenotype can be changed include barley, rye, millet, sorghum, currant, avocado, citrus fruits such as oranges, lemons, grapefruit and tangerines, artichoke, cherries, nuts such as the walnut and peanut, endive, leek, roots, such as arrowroot, beet, cassava, turnip, radish, yam, and sweet potato, and beans. The homologous sequences may also be derived from woody species, such pine, poplar and eucalyptus.

Transcription factors that are homologous to the listed sequences will typically share at least about 30% amino acid sequence identity. More closely related transcription factors can share at least about 50%, about 60%, about 65%, about 70%, about 75% or about 80% or about 90% or about 95% or about 98% or more sequence identity with the listed sequences. Factors that are most closely related to the listed sequences share, e.g., at least about 85%, about 90% or about 95% or more % sequence identity to the listed sequences. At the nucleotide level, the sequences will typically share at least about 40% nucleotide sequence identity, preferably at least about 50%, about 60%, about 70% or about 80% sequence identity, and more preferably about 85%, about 90%, about 95% or about 97% or more sequence identity to one or more of the listed sequences. The degeneracy of the genetic code enables major variations in the nucleotide sequence of a polynucleotide while maintaining the amino acid sequence of the encoded protein. Conserved domains within a transcription factor family may exhibit a higher degree of sequence homology, such as at least 65% sequence identity including conservative substitutions, and preferably at least 80% sequence identity.

### Identifying Nucleic Acids by Hybridization

Polynucleotides homologous to the sequences illustrated in the Sequence Listing can be identified, e.g., by hybridization to each other under stringent or under highly stringent conditions. Single stranded polynucleotides hybridize when they associate based on a variety of well characterized physico-chemical forces, such as hydrogen bonding, solvent exclusion, base stacking and the like. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number), as described in more detail in the references cited above.

An example of stringent hybridization conditions for hybridization of complementary nucleic acids which have more than 100 complementary residues on a filter in a Southern or northern blot is about 5°C to 20°C lower than the thermal melting point (Tm) for the specific sequence at a defined ionic strength and pH. The Tₘ is the temperature (under defined ionic strength and pH) at which 50% of the target sequence hybridizes to a perfectly matched probe. Nucleic acid molecules that hybridize under stringent conditions will typically hybridize to a probe based on either the entire cDNA or selected portions, e.g., to a unique subsequence, of the cDNA under wash conditions of 0.2x SSC to 2.0 x SSC, 0.1% SDS at 50-65° C, for example 0.2 x SSC, 0.1% SDS at 65°C. For identification of less closely related homologues washes can be performed at a lower temperature, e.g., 50° C. In general, stringency is increased by raising the wash temperature and/or decreasing the concentration of SSC.

As another example, stringent conditions can be selected such that an oligonucleotide that is perfectly complementary to the coding oligonucleotide hybridizes to the coding oligonucleotide with at least about a 5-10x higher signal to noise ratio than the ratio for hybridization of the perfectly complementary oligonucleotide to a nucleic acid encoding a transcription factor known as of the filing date of the application. Conditions can be selected such that a higher signal to noise ratio is observed in the particular assay which is used, e.g., about 15x, 25x, 35x, 50x or more. Accordingly, the subject nucleic acid hybridizes to the unique coding oligonucleotide with at least a 2x higher signal to noise ratio as compared to hybridization of the coding oligonucleotide to a nucleic acid encoding known polypeptide. Again, higher signal to noise ratios can be selected, e.g., about 5x, 10x, 25x, 35x, 50x or more. The particular signal will depend on the label used in the relevant assay, e.g., a fluorescent label, a colorimetric label, a radioactive label, or the like.

Alternatively, transcription factor homologue polypeptides can be obtained by screening an expression library using antibodies specific for one or more transcription factors. With the provision herein of the disclosed transcription factor, and transcription factor homologue nucleic acid sequences, the encoded polypeptide(s) can be expressed and purified in a heterologous expression system (e.g., *E*. *coli*) and used to raise antibodies (monoclonal or polyclonal) specific for the polypeptide(s) in question. Antibodies can also be raised against synthetic peptides derived from transcription factor, or transcription factor homologue, amino acid sequences. Methods of raising antibodies are well known in the art and are described in Harlow and Lane (1988) Antibodies: A Laboratory Manual, Cold Spring Harbor Laboratory, New York. Such antibodies can then be used to screen an expression library produced from the plant from which it is desired to clone additional transcription factor homologues, using the methods described above. The selected cDNAs can be confirmed by sequencing and enzymatic activity.

### SEQUENCE VARIATIONS

It will readily be appreciated by those of skill in the art, that any of a variety of polynucleotide sequences are capable of encoding the transcription factors and transcription factor homologue polypeptide described herein. Due to the degeneracy of the genetic code, many different polynucleotides can encode identical and/or substantially similar polypeptides in addition to those sequences illustrated in the Sequence Listing.

For example, Table 1 illustrates, e.g., that the codons AGC, AGT, TCA, TCC, TCG, and TCT all encode the same amino acid: serine. Accordingly, at each position in the sequence where there is a codon encoding serine, any of the above trinucleotide sequences can be used without altering the encoded polypeptide.

**Table 1**

| Amino acids | | | Codon | | | | | |
|---|---|---|---|---|---|---|---|---|
| Alanine | Ala | A | GCA | GCC | GCG | GCU | | |
| Cysteine | Cys | C | TGC | TGT | | | | |
| Aspartic acid | Asp | D | GAC | GAT | | | | |
| Glutamic acid | Glu | E | GAA | GAG | | | | |
| Phenylalanine | Phe | F | TTC | TTT | | | | |
| Glycine | Gly | G | GGA | GGC | GGG | GGT | | |
| Histidine | His | H | CAC | CAT | | | | |
| Isoleucine | Ile | I | ATA | ATC | ATT | | | |
| Lysine | Lys | K | AAA | AAG | | | | |
| Leucine | Leu | L | TTA | TTG | CTA | CTC | CTG | CTT |
| Methionine | Met | M | ATG | | | | | |
| Asparagine | Asn | N | AAC | AAT | | | | |
| Proline | Pro | P | CCA | CCC | CCG | CCT | | |
| Glutamine | Gln | Q | CAA | CAG | | | | |
| Arginine | Arg | R | AGA | AGG | CGA | CGC | CGG | CGT |
| Serine | Ser | S | AGC | AGT | TCA | TCC | TCG | TCT |
| Threonine | Thr | T | ACA | ACC | ACG | ACT | | |
| Valine | Val | V | GTA | GTC | GTG | GTT | | |
| Tryptophan | Trp | W | TGG | | | | | |
| Tyrosine | Tyr | Y | TAC | TAT | | | | |

Sequence alterations that do not change the amino acid sequence encoded by the polynucleotide are termed "silent" variations. With the exception of the codons ATG and TGG, encoding methionine and tryptophan, respectively, any of the possible codons for the same amino acid can be substituted by a variety of techniques, e.g., site-directed mutagenesis, available in the art. Accordingly, any and all such variations of a sequence selected from the above table are a feature of the invention.

In addition to silent variations, other conservative variations that alter one, or a few amino acids in the encoded polypeptide, can be made without altering the function of the polypeptide.

For example, substitutions, deletions and insertions introduced into the sequences provided in the Sequence Listing are also described. Such sequence modifications can be engineered into a sequence by site-directed mutagenesis (Wu (ed.) Meth. Enzymol. (1993) vol. 217, Academic Press) or the other methods noted below. Amino acid substitutions are typically of single residues; insertions usually will be on the order of about from 1 to 10 amino acid residues; and deletions will range about from 1 to 30 residues. In preferred embodiments, deletions or insertions are made in adjacent pairs, e.g., a deletion of two residues or insertion of two residues. Substitutions, deletions, insertions or any combination thereof can be combined to arrive at a sequence. The mutations that are made in the polynucleotide encoding the transcription factor should not place the sequence out of reading frame and should not create complementary regions that could produce secondary mRNA structure. Preferably, the polypeptide encoded by the DNA performs the desired function.

Conservative substitutions are those in which at least one residue in the amino acid sequence has been removed and a different residue inserted in its place. Such substitutions generally are made in accordance with the Table 2 when it is desired to maintain the activity of the protein. Table 2 shows amino acids which can be substituted for an amino acid in a protein and which are typically regarded as conservative substitutions.

**Table 2**

| **Residue** | **Conservative Substitutions** |
|---|---|
| Ala | Ser |
| Arg | Lys |
| Asn | Gln; His |
| Asp | Glu |
| Gln | Asn |
| Cys | Ser |
| Glu | Asp |
| Gly | Pro |
| His | Asn; Gln |
| Ile | Leu, Val |
| Leu | Ile; Val |
| Lys | Arg; Gln |
| Met | Leu; Ile |
| Phe | Met; Leu; Tyr |
| Ser | Thr; Gly |
| Thr | Ser;Val |
| Trp | Tyr |
| Tyr | Trp; Phe |
| Val | Ile; Leu |

Substitutions that are less conservative than those in Table 2 can be selected by picking residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions which in general are expected to produce the greatest changes in protein properties will be those in which (a) a hydrophilic residue, e.g., seryl or threonyl, is substituted for (or by) a hydrophobic residue, e.g., leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine.

### FURTHER MODIFYING SEQUENCES MUTATION/ FORCED EVOLUTION

In addition to generating silent or conservative substitutions as noted, above, the present specification optionally includes methods of modifying the sequences of the Sequence Listing. In the methods, nucleic acid or protein modification methods are used to alter the given sequences to produce new sequences and/or to chemically or enzymatically modify given sequences to change the properties of the nucleic acids or proteins.

Thus, in one embodiment, given nucleic acid sequences are modified, e.g., according to standard mutagenesis or artificial evolution methods to produce modified sequences. For example, Ausubel, *supra,* provides additional details on mutagenesis methods. Artificial forced evolution methods are described, e.g., by Stemmer (1994) Nature 370:389-391, and Stemmer (1994) Proc. Natl. Acad. Sci. USA 91:10747-10751. Many other mutation and evolution methods are also available and expected to be within the skill of the practitioner.

Similarly, chemical or enzymatic alteration of expressed nucleic acids and polypeptides can be performed by standard methods. For example, sequence can be modified by addition of lipids, sugars, peptides, organic or inorganic compounds, by the inclusion of modified nucleotides or amino acids, or the like. For example, protein modification techniques are illustrated in Ausubel, *supra.* Further details on chemical and enzymatic modifications can be found herein. These modification methods can be used to modify any given sequence, or to modify any sequence produced by the various mutation and artificial evolution modification methods noted herein.

Accordingly, described herein is the modification of any given nucleic acid by mutation, evolution, chemical or enzymatic modification, or other available methods, as well as for the products produced by practicing such methods, e.g., using the sequences herein as a starting substrate for the various modification approaches.

For example, optimized coding sequence containing codons preferred by a particular prokaryotic or eukaryotic host can be used e.g., to increase the rate of translation or to produce recombinant RNA transcripts having desirable properties, such as a longer half-life, as compared with transcripts produced using a non-optimized sequence. Translation stop codons can also be modified to reflect host preference. For example, preferred stop codons for *S*. *cerevisiae* and mammals are TAA and TGA, respectively. The preferred stop codon for monocotyledonous plants is TGA, whereas insects and *E. coli* prefer to use TAA as the stop codon.

The polynucleotide sequences can also be engineered in order to alter a coding sequence for a variety of reasons, including but not limited to, alterations which modify the sequence to facilitate cloning, processing and/or expression of the gene product. For example, alterations are optionally introduced using techniques which are well known in the art, e.g., site-directed mutagenesis, to insert new restriction sites, to alter glycosylation patterns, to change codon preference, to introduce splice sites, etc.

Furthermore, a fragment or domain derived from any of the polypeptides can be combined with domains derived from other transcription factors or synthetic domains to modify the biological activity of a transcription factor. For instance, a DNA binding domain derived from a transcription factor of the invention can be combined with the activation domain of another transcription factor or with a synthetic activation domain. A transcription activation domain assists in initiating transcription from a DNA binding site. Examples include the transcription activation region of VP 16 or GAL4 (Moore et al. (1998) Proc. Natl. Acad. Sci. USA 95: 376-381; and Aoyama et al. (1995) Plant Cell 7:1773-1785), peptides derived from bacterial sequences (Ma and Ptashne (1987) Cell 51; 113-119) and synthetic peptides (Giniger and Ptashne, (1987) Nature 330:670-672).

### EXPRESSION AND MODIFICATION OF POLYPEPTIDES

Typically, polynucleotide sequences are incorporated into recombinant DNA (or RNA) molecules that direct expression of polypeptides in appropriate host cells, transgenic plants, in vitro translation systems, or the like. Due to the inherent degeneracy of the genetic code, nucleic acid sequences which encode substantially the same or a functionally equivalent amino acid sequence can be substituted for any listed sequence to provide for cloning and expressing the relevant homologue.

### Vectors, Promoters and Expression Systems

Described herein are recombinant constructs comprising one or more of the nucleic acid sequences herein. The constructs typically comprise a vector, such as a plasmid, a cosmid, a phage, a virus (e.g., a plant virus), a bacterial artificial chromosome (BAC), a yeast artificial chromosome (YAC), or the like, into which a nucleic acid sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available.

General texts which describe molecular biological techniques useful herein, including the use and production of vectors, promoters and many other relevant topics, include Berger, Sambrook and *Ausubel, supra.* Any of the identified sequences can be incorporated into a cassette or vector, e.g., for expression in plants. A number of expression vectors suitable for stable transformation of plant cells or for the establishment of transgenic plants have been described including those described in Weissbach and Weissbach, (1989) Methods for Plant Molecular Biology, Academic Press, and Gelvin et al., (1990) Plant Molecular Biology Manual, Kluwer Academic Publishers. Specific examples include those derived from a Ti plasmid of *Agrobacterium tumefaciens,* as well as those disclosed by Herrera-Estrella et al. (1983) Nature 303: 209, Bevan (1984) Nucl Acid Res. 12: 8711-8721, Klee (1985) Bio/lTechnology 3: 637-642, for dicotyledonous plants.

Alternatively, non-Ti vectors can be used to transfer the DNA into monocotyledonous plants and cells by using free DNA delivery techniques. Such methods can involve, for example, the use of liposomes, electroporation, microprojectile bombardment, silicon carbide whiskers, and viruses. By using these methods transgenic plants such as wheat, rice (Christou (1991) Bio/Technology 9: 957-962) and corn (Gordon-Kamm (1990) Plant Cell 2: 603-618) can be produced. An immature embryo can also be a good target tissue for monocots for direct DNA delivery techniques by using the particle gun (Weeks et al. (1993) Plant Physiol 102: 1077-1084; Vasil (1993) Bio/fechnology 10: 667-674; Wan and Lemeaux (1994) Plant Physiol 104: 37-48, and for Agrobacterium-mediated DNA transfer (Ishida et al. (1996) Nature Biotech 14: 745-750).

Typically, plant transformation vectors include one or more cloned plant coding sequence (genomic or cDNA) under the transcriptional control of 5' and 3' regulatory sequences and a dominant selectable marker. Such plant transformation vectors typically also contain a promoter (e.g., a regulatory region controlling inducible or constitutive, environmentally-or developmentally-regulated, or cell- or tissue-specific expression), a transcription initiation start site, an RNA processing signal (such as intron splice sites), a transcription termination site, and/or a polyadenylation signal.

Examples of constitutive plant promoters which can be useful for expressing the TF sequence include: the cauliflower mosaic virus (CaMV) 35S promoter, which confers constitutive, high-level expression in most plant tissues (*see,* e.g., Odel et al. (1985) Nature 313:810); the nopaline synthase promoter (An et al. (1988) Plant Physiol 88:547); and the octopine synthase promoter (Fromm et al. (1989) Plant Cell 1: 977).

A variety of plant gene promoters that regulate gene expression in response to environmental, hormonal, chemical, developmental signals, and in a tissue-active manner can be used for expression of a TF sequence in plants. Choice of a promoter is based largely on the phenotype of interest and is determined by such factors as tissue (e.g., seed, fruit, root, pollen, vascular tissue, flower, carpel, etc.), inducibility (e.g., in response to wounding, heat, cold, drought, light, pathogens, etc.), timing, developmental stage, and the like. Numerous known promoters have been characterized and can favorable be employed to promote expression of a polynucleotide of the invention in a transgenic plant or cell of interest. For example, tissue specific promoters include: seed-specific promoters (such as the napin, phaseolin or DC3 promoter described in US Pat. No. 5,773,697), fruit-specific promoters that are active during fruit ripening (such as the dru 1 promoter (US Pat. No. 5,783,393), or the 2A11 promoter (US Pat. No. 4,943,674) and the tomato polygalacturonase promoter (Bird et al. (1988) Plant Mol Biol 11:651), root-specific promoters, such as those disclosed in US Patent Nos. 5,618,988, 5,837,848 and 5,905,186, pollen-active promoters such as PTA29, PTA26 and PTA13 (US Pat. No. 5,792,929), promoters active in vascular tissue (Ringli and Keller (1998) Plant Mol Biol 37:977-988), flowerspecific (Kaiser et al, (1995) Plant Mol Biol 28:231-243), pollen (Baerson et al. (1994) Plant Mol Biol 26:1947-1959), carpels (Ohl et al. (1990) Plant Cell 2:837-848), pollen and ovules (Baerson et al. (1993) Plant Mol Biol 22:255-267), auxin-inducible promoters (such as that described in van der Kop et al. (1999) Plant Mol Biol 39:979-990 or Baumann et al. (1999) Plant Cell 11:323-334), cytokinin-inducible promoter (Guevara-Garcia (1998) Plant Mol Biol 38:743-753), promoters responsive to gibberellin (Shi et al. (1998) Plant Mol Biol 38:1053-1060, Willmott et al. (1998) 38:817-825) and the like. Additional promoters are those that elicit expression in response to heat (Ainley et al. (1993) Plant Mol Biol 22: 13-23), light (e.g., the pea rbcS-3A promoter, Kuhlemeier et al. (1989) Plant Cell 1:471, and the maize rbcS promoter, Schaffner and Sheen (1991) Plant Cell 3: 997); wounding (e.g., *wunI,* Siebertz et al. (1989) Plant Cell 1: 961); pathogens (such as the PR-1 promoter described in Buchel et al. (1999) Plant Mol. Biol. 40:387-396, and the PDF1.2 promoter described in Manners et al. (1998) Plant Mol. Biol. 38:1071-80), and chemicals such as methyl jasmonate or salicylic acid (Gatz et al. (1997) Plant Mol Biol 48: 89-108). In addition, the timing of the expression can be controlled by using promoters such as those acting at senescence (An and Amazon (1995) Science 270: 1986-1988); or late seed development (Odell et al. (1994) Plant Physiol 106:447-458).

Plant expression vectors can also include RNA processing signals that can be positioned within, upstream or downstream of the coding sequence. In addition, the expression vectors can include additional regulatory sequences from the 3'-untranslated region of plant genes, e.g., a 3' terminator region to increase mRNA stability of the mRNA, such as the PI-II terminator region of potato or the octopine or nopaline synthase 3' terminator regions.

### Additional Expression Elements

Specific initiation signals can aid in efficient translation of coding sequences. These signals can include, e.g., the ATG initiation codon and adjacent sequences. In cases where a coding sequence, its initiation codon and upstream sequences are inserted into the appropriate expression vector, no additional translational control signals may be needed. However, in cases where only coding sequence (e.g., a mature protein coding sequence), or a portion thereof, is inserted, exogenous transcriptional control signals including the ATG initiation codon can be separately provided. The initiation codon is provided in the correct reading frame to facilitate transcription. Exogenous transcriptional elements and initiation codons can be of various origins, both natural and synthetic. The efficiency of expression can be enhanced by the inclusion of enhancers appropriate to the cell system in use.

### Expression Hosts

The present specification also relates to host cells which are transduced with vectors of the invention, and the production of polypeptides described herein (including fragments thereof) by recombinant techniques. Host cells are genetically engineered (i.e, nucleic acids are introduced, e.g., transduced, transformed or transfected) with the vectors described herein, which may be, for example, a cloning vector or an expression vector comprising the relevant nucleic acids herein. The vector is optionally a plasmid, a viral particle, a phage, a naked nucleic acids, *etc*. The engineered host cells can be cultured in conventional nutrient media modified as appropriate for activating promoters, selecting transformants, or amplifying the relevant gene. The culture conditions, such as temperature, pH and the like, are those previously used with the host cell selected for expression, and will be apparent to those skilled in the art and in the references cited herein, including, Sambrook and Ausubel.

The host cell can be a eukaryotic cell, such as a yeast cell, or a plant cell, or the host cell can be a prokaryotic cell, such as a bacterial cell. Plant protoplasts are also suitable for some applications. For example, the DNA fragments are introduced into plant tissues, cultured plant cells or plant protoplasts by standard methods including electroporation (Fromm et al., (1985) Proc. Natl. Acad. Sci. USA 82, 5824, infection by viral vectors such as cauliflower mosaic virus (CaMV) (Hohn et al., (1982) Molecular Biology of Plant Tumors, (Academic Press, New York) pp. 549-560; US 4,407,956), high velocity ballistic penetration by small particles with the nucleic acid either within the matrix of small beads or particles, or on the surface (Klein et al., (1987) Nature 327, 70-73), use of pollen as vector (WO 85/01856), or use of *Agrobacterium tumefaciens* or *A. rhizogenes* carrying a T-DNA plasmid in which DNA fragments are cloned. The T-DNA plasmid is transmitted to plant cells upon infection by *Agrobacterium tumefaciens,* and a portion is stably integrated into the plant genome (Horsch et al. (1984) Science 233:496-498; Fraley et al. (1983) Proc. Natl. Acad. Sci. USA 80, 4803).

The cell can include a nucleic acid described herein which encodes a polypeptide, wherein the cells expresses a polypeptide of the invention. The cell can also include vector sequences, or the like.. Furthermore, cells and transgenic plants which include any polypeptide or nucleic acid above or throughout this specification, e.g., produced by transduction of a vector described herein are an additional feature of the specification.

For long-term, high-yield production of recombinant proteins, stable expression can be used. Host cells transformed with a nucleotide sequence encoding a polypeptide described herein are optionally cultured under conditions suitable for the expression and recovery of the encoded protein from cell culture. The protein or fragment thereof produced by a recombinant cell may be secreted, membrane-bound, or contained intracellularly, depending on the sequence and/or the vector used. As will be understood by those of skill in the art, expression vectors containing polynucleotides encoding mature proteins of the invention can be designed with signal sequences which direct secretion of the mature polypeptides through a prokaryotic or eukaryotic cell membrane.

### Modified Amino Acids

Polypeptides of the invention may contain one or more modified amino acids. The presence of modified amino acids may be advantageous in, for example, increasing polypeptide half-life, reducing polypeptide antigenicity or toxicity, increasing polypeptide storage stability, or the like: Amino acid(s) are modified, for example, co-translationally or post-translationally during recombinant production or modified by synthetic or chemical means.

Non-limiting examples of a modified amino acid include incorporation or other use of acetylated amino acids, glycosylated amino acids, sulfated amino acids, prenylated (e.g., farnesylated, geranylgeranylated) amino acids, PEG modified (e.g., "PEGylated") amino acids, biotinylated amino acids, carboxylated amino acids, phosphorylated amino acids, etc. References adequate to guide one of skill in the modification of amino acids are replete throughout the literature.

### IDENTIFICATION OF ADDITIONAL FACTORS

A transcription factor provided herein can also be used to identify additional endogenous or exogenous molecules that can affect a phentoype or trait of interest. On the one hand, such molecules include organic (small or large molecules) and/or inorganic compounds that affect expression of (i.e., regulate) a particular transcription factor. Alternatively, such molecules include endogenous molecules that are acted upon either at a transcriptional level by a transcription factor described herein to modify a phenotype as desired. For example, the transcription factors can be employed to identify one or more downstream gene with which is subject to a regulatory effect of the transcription factor. In one approach, a transcription factor or transcription factor homologue described herein is expressed in a host cell, e.g, a transgenic plant cell, tissue or explant, and expression products, either RNA or protein, of likely or random targets are monitored, e.g., by hybridization to a microarray of nucleic acid probes corresponding to genes expressed in a tissue or cell type of interest, by two-dimensional gel electrophoresis of protein products, or by any other method known in the art for assessing expression of gene products at the level of RNA or protein. Alternatively, a transcription factor can be used to identify promoter sequences (i.e., binding sites) involved in the regulation of a downstream target. After identifying a promoter sequence, interactions between the transcription factor and the promoter sequence can be modified by changing specific nucleotides in the promoter sequence or specific amino acids in the transcription factor that interact with the promoter sequence to alter a plant trait. Typically, transcription factor DNA binding sites are identified by gel shift assays. After identifying the promoter regions, the promoter region sequences can be employed in double-stranded DNA arrays to identify molecules that affect the interactions of the transcription factors with their promoters (Bulyk et al. (1999) Nature Biotechnology 17:573-577).

The identified transcription factors are also useful to identify proteins that modify the activity of the transcription factor. Such modification can occur by covalent modification, such as by phosphorylation, or by protein-protein (homo or-heteropolymer) interactions. Any method suitable for detecting protein-protein interactions can be employed. Among the methods that can be employed are co-immunoprecipitation, cross-linking and co-purification through gradients or chromatographic columns, and the two-hybrid yeast system.

The two-hybrid system detects protein interactions in vivo and is described in Chien, et al., (1991), Proc. Natl. Acad. Sci. USA 88, 9578-9582 and is commercially available from Clontech (Palo Alto, Calif.). In such a system, plasmids are constructed that encode two hybrid proteins: one consists of the DNA-binding domain of a transcription activator protein fused to the TF polypeptide and the other consists of the transcription activator protein's activation domain fused to an unknown protein that is encoded by a cDNA that has been recombined into the plasmid as part of a cDNA library. The DNA-binding domain fusion plasmid and the cDNA library are transformed into a strain of the yeast *Saccharomyces cerevisiae* that contains a reporter gene (e.g., lacZ) whose regulatory region contains the transcription activator's binding site. Either hybrid protein alone cannot activate transcription of the reporter gene. Interaction of the two hybrid proteins reconstitutes the functional activator protein and results in expression of the reporter gene, which is detected by an assay for the reporter gene product. Then, the library plasmids responsible for reporter gene expression are isolated and sequenced to identify the proteins encoded by the library plasmids. After identifying proteins that interact with the transcription factors, assays for compounds that interfere with the TF protein-protein interactions can be preformed.

### IDENTIFICATION OF MODULATORS

In addition to the intracellular molecules described above, extracellular molecules that alter activity or expression of a transcription factor, either directly or indirectly, can be identified. For example, the methods can entail first placing a candidate molecule in contact with a plant or plant cell. The molecule can be introduced by topical administration, such as spraying or soaking of a plant, and then the molecule's effect on the expression or activity of the TF polypeptide or the expression of the polynucleotide monitored. Changes in the expression of the TF polypeptide can be monitored by use of polyclonal or monoclonal antibodies, gel electrophoresis or the like. Changes in the expression of the corresponding polynucleotide sequence can be detected by use of microarrays, Northern, quantitative PCR, or any other technique for monitoring changes in mRNA expression. These techniques are exemplified in Ausubel et al. (eds) Current Protocols in Molecular Biology, John Wiley & Sons (1998). Such changes in the expression levels can be correlated with modified plant traits and thus identified molecules can be useful for soaking or spraying on fruit, vegetable and grain crops to modify traits in plants.

Essentially any available composition can be tested for modulatory activity of expression or activity of any nucleic acid or polypeptide herein. Thus, available libraries of compounds such as chemicals, polypeptides, nucleic acids and the like can be tested for modulatory activity. Often, potential modulator compounds can be dissolved in aqueous or organic (e.g., DMSO-based) solutions for easy delivery to the cell or plant of interest in which the activity of the modulator is to be tested. Optionally, the assays are designed to screen large modulator composition libraries by automating the assay steps and providing compounds from any convenient source to assays, which are typically run in parallel (e.g., in microtiter formats on microtiter plates in robotic assays).

In one embodiment, high throughput screening methods involve providing a combinatorial library containing a large number of potential compounds (potential modulator compounds). Such "combinatorial chemical libraries" are then screened in one or more assays, as described herein, to identify those library members (particular chemical species or subclasses) that display a desired characteristic activity. The compounds thus identified can serve as target compounds.

A combinatorial chemical library can be, e.g., a collection of diverse chemical compounds generated by chemical synthesis or biological synthesis. For example, a combinatorial chemical library such as a polypeptide library is formed by combining a set of chemical building blocks (e.g., in one example, amino acids) in every possible way for a given compound length (i.e., the number of amino acids in a polypeptide compound of a set length). Exemplary libraries include peptide libraries, nucleic acid libraries, antibody libraries (see, e.g., Vaughn et al. (1996) Nature Biotechnology, 14(3):309-314 and WO 97/00271, carbohydrate libraries (see, e.g., Liang et al. Science (1996) 274:1520-1522 and U.S. Patent 5,593,853), peptide nucleic acid libraries (see, e.g., U.S. Patent 5,539,083), and small organic molecule libraries (see, e.g., benzodiazepines, Baum C&EN Jan 18, page 33 (1993); isoprenoids, U.S. Patent 5,569,588; thiazolidinones and metathiazanones, U.S. Patent 5,549,974; pyrrolidines, U.S. Patents 5,525,735 and 5,519,134; morpholino compounds, U.S. Patent 5,506,337) and the like.

Preparation and screening of combinatorial or other libraries is well known to those of skill in the art. Such combinatorial chemical libraries include, but are not limited to, peptide libraries (see, e.g., U.S. Patent 5,010,175, Furka, Int. J. Pept. Prot. Res. 37:487-493 (1991) and Houghton et al. Nature 354:84-88 (1991)). Other chemistries for generating chemical diversity libraries can also be used.

In addition, as noted, compound screening equipment for high-throughput screening is generally available, e.g., using any of a number of well known robotic systems that have also been developed for solution phase chemistries useful in assay systems. These systems include automated workstations including an automated synthesis apparatus and robotic systems utilizing robotic arms. Any of the above devices are suitable for high-throughput screening of potential modulators. The nature and implementation of modifications to these devices (if any) so that they can operate as discussed herein will be apparent to persons skilled in the relevant art.

Indeed, entire high throughput screening systems are commercially available. These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization. Similarly, microfluidic implementations of screening are also commercially available.

The manufacturers of such systems provide detailed protocols the various high throughput. Thus, for example, Zymark Corp. provider technical bulletins describing screening systems for detecting the modulation of gene transcription, ligand binding, and the like. The integrated systems herein, in addition to providing for sequence alignment and, optionally, synthesis of relevant nucleic acids, can include such screening apparatus to identify modulators that have an effect on one or more polynucleotides or polypeptides described herein.

In some assays it is desirable to have positive controls to ensure that the components of the assays are working properly. At least two types of positive controls are appropriate. That is, known transcriptional activators or inhibitors can be incubated with cells/plants/ etc. in one sample of the assay, and the resulting increase/decrease in transcription can be detected by measuring the resulting increase in RNA/ protein expression, etc., according to the methods herein. It will be appreciated that modulators can also be combined with transcriptional activators or inhibitors to find modulators which inhibit transcriptional activation or transcriptional repression. Either expression of the nucleic acids and proteins herein or any additional nucleic acids or proteins activated by the nucleic acids or proteins herein, or both, can be monitored.

Described herein is a method for identifying compositions that modulate the activity or expression of a polynucleotide or polypeptide of the disclosure. For example, a test compound, whether a small or large molecule, is placed in contact with a cell, plant (or plant tissue or explant), or composition comprising the polynucleotide or polypeptide of interest and a resulting effect on the cell, plant, (or tissue or explant) or composition is evaluated by monitoring, either directly or indirectly, one or more of: expression level of the polynucleotide or polypeptide, activity (or modulation of the activity) of the polynucleotide or polypeptide. In some cases, an alteration in a plant phenotype can be detected following contact of a plant (or plant cell, or tissue or explant) with the putative modulator, e.g., by modulation of expression or activity of a polynucleotide or polypeptide described herein.

### SUBSEQUENCES

Also contemplated are uses of polynucleotides, also referred to herein as oligonucleotides, typically having at least 12 bases, preferably at least 15, more preferably at least 20, 30, or 50 bases, which hybridize under at least highly stringent (or ultra-high stringent or ultra-ultra- high stringent conditions) conditions to a polynucleotide sequence described above. The polynucleotides may be used as probes, primers, sense and antisense agents, and the like, according to methods as noted *supra.*

Subsequences of the polynucleotides described herein, including polynucteotide fragments and oligonucleotides are useful as nucleic acid probes and primers. An oligonucleotide suitable for use as a probe or primer is at least about 15 nucleotides in length, more often at least about 18 nucleotides, often at least about 21 nucleotides, frequently at least about 30 nucleotides, or about 40 nucleotides, or more in length. A nucleic acid probe is useful in hybridization protocols, e.g., to identify additional polypeptide homologues of the invention, including protocols for microarray experiments. Primers can be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, e.g., by the polymerase chain reaction (PCR) or other nucleic-acid amplification methods. See Sambrook and Ausubel, *supra.*

In addition, the invention includes an isolated or recombinant polypeptide including a subsequence of at least about 15 contiguous amino acids encoded by the recombinant or isolated polynucleotides of the invention. For example, such polypeptides, or domains or fragments thereof, can be used as immunogens, e.g., to produce antibodies specific for the polypeptide sequence, or as probes for detecting a sequence of interest. A subsequence can range in size from about 15 amino acids in length up to and including the full length of the polypeptide.

### PRODUCTION OF TRANSGENIC PLANTS

### Modification of Traits

The polynucleotides described herein are favorably employed to produce transgenic plants with various traits, or characteristics, that have been modified in a desirable manner, e.g., to improve the seed characteristics of a plant. For example, alteration of expression levels or patterns (e.g., spatial or temporal expression patterns) of one or more of the transcription factors (or transcription factor homologues) described herein, as compared with the levels of the same protein found in a wild type plant, can be used to modify a plant's traits. An illustrative example of trait modification, improved biochemical characteristics, by altering expression levels of a particular transcription factor is described further in the Examples and the Sequence Listing.

### Antisense and Cosuppression Approaches

In addition to expression of the nucleic acids described herein as gene replacement or plant phenotype modification nucleic acids, the nucleic acids are also useful for sense and antisense suppression of expression, e.g., to down-regulate expression of a nucleic acid of the invention, e.g., as a further mechanism for modulating plant phenotype. That is, the nucleic acids , or subsequences or anti-sense sequences thereof, can be used to block expression of naturally occurring homologous nucleic acids. A variety of sense and anti-sense technologies are known in the art, e.g., as set forth in Lichtenstein and Nellen (1997) Antisense Technology: A Practical Approach IRL Press at Oxford University, Oxford, England. In general, sense or antisense sequences are introduced into a cell, where they are optionally amplified, e.g., by transcription. Such sequences include both simple oligonucleotide sequences and catalytic sequences such as ribozymes.

For example, a reduction or elimination of expression (i.e., a "knock-out") of a transcription factor or transcription factor homologue polypeptide in a transgenic plant, e.g., to modify a plant trait, can be obtained by introducing an antisense construct corresponding to the polypeptide of interest as a cDNA. For antisense suppression, the transcription factor or homologue cDNA is arranged in reverse orientation (with respect to the coding sequence) relative to the promoter sequence in the expression vector. The introduced sequence need not be the full length cDNA or gene, and need not be identical to the cDNA or gene found in the plant type to be transformed. Typically, the antisense sequence need only be capable of hybridizing to the target gene or RNA of interest. Thus, where the introduced sequence is of shorter length, a higher degree of homology to the endogenous transcription factor sequence will be needed for effective antisense suppression. While antisense sequences of various lengths can be utilized, preferably, the introduced antisense sequence in the vector will be at least 30 nucleotides in length, and improved antisense suppression will typically be observed as the length of the antisense sequence increases. Preferably, the length of the antisense sequence in the vector will be greater than 100 nucleotides. Transcription of an antisense construct as described results in the production of RNA molecules that are the reverse complement ofmRNA molecules transcribed from the endogenous transcription factor gene in the plant cell.

Suppression of endogenous transcription factor gene expression can also be achieved using a ribozyme. Ribozymes are RNA molecules that possess highly specific endoribonuclease activity. The production and use of ribozymes are disclosed in U.S. Patent No. 4,987,071 and U.S. Patent No. 5,543,508. Synthetic ribozyme sequences including antisense RNAs can be used to confer RNA cleaving activity on the antisense RNA, such that endogenous mRNA molecules that hybridize to the antisense RNA are cleaved, which in turn leads to an enhanced antisense inhibition of endogenous gene expression.

Vectors in which RNA encoded by a transcription factor or transcription factor homologue cDNA is over-expressed can also be used to obtain co-suppression of a corresponding endogenous gene, e.g., in the manner described in U.S. Patent No. 5,231,020 to Jorgensen. Such co-suppression (also termed sense suppression) does not require that the entire transcription factor cDNA be introduced into the plant cells, nor does it require that the introduced sequence be exactly identical to the endogenous transcription factor gene of interest. However, as with antisense suppression, the suppressive efficiency will be enhanced as specificity of hybridization is increased, e.g., as the introduced sequence is lengthened, and/or as the sequence similarity between the introduced sequence and the endogenous transcription factor gene is increased.

Vectors expressing an untranslatable form of the transcription factor mRNA, e.g., sequences comprising one or more stop codon, or nonsense mutation) can also be used to suppress expression of an endogenous transcription factor, thereby reducing or eliminating it's activity and modifying one or more traits. Methods for producing such constructs are described in U.S. Patent No. 5,583,021. Preferably, such constructs are made by introducing a premature stop codon into the transcription factor gene. Alternatively, a plant trait can be modified by gene silencing using double-strand RNA (Sharp (1999) Genes and Development 13: 139-141).

Another method for abolishing the expression of a gene is by insertion mutagenesis using the T-DNA of *Agrobacterium tumefaciens.* After generating the insertion mutants, the mutants can be screened to identify those containing the insertion in a transcription factor or transcription factor homologue gene. Plants containing a single transgene insertion event at the desired gene can be crossed to generate homozygous plants for the mutation (Koncz et al. (1992) Methods in Arabidopsis Research, World Scientific).

Alternatively, a plant phenotype can: be altered by eliminating an endogenous gene, such as a transcription factor or transcription factor homologue, e.g., by homologous recombination (Kempin et al. (1997) Nature 389:802).

A plant trait can also be modified by using the cre-lox system (for example, as described in US Pat. No. 5,658,772). A plant genome can be modified to include first and second lox sites that are then contacted with a Cre recombinase. If the lox sites are in the same orientation, the intervening DNA sequence between the two sites is excised. If the lox sites are in the opposite orientation, the intervening sequence is inverted.

The polynucleotides and polypeptides of this invention can also be expressed in a plant in the absence of an expression cassette by manipulating the activity or expression level of the endogenous gene by other means. For example, by ectopically expressing a gene by T-DNA activation tagging (Ichikawa et al. (1997) Nature 390 698-701; Kakimoto et al. (1996) Science 274: 982-985). This method entails transforming a plant with a gene tag containing multiple transcriptional enhancers and once the tag has inserted into the genome, expression of a flanking gene coding sequence becomes deregulated. In another example, the transcriptional machinery in a plant can be modified so as to increase transcription levels of a polynucleotide of the invention (*See*, e.g., PCT Publications WO 96/06166 and WO 98/53057 which describe the modification of the DNA binding specificity of zinc finger proteins by changing particular amino acids in the DNA binding motif).

The transgenic plant can also include the machinery necessary for expressing or altering the activity of a polypeptide encoded by an endogenous gene, for example by altering the phosphorylation state of the polypeptide to maintain it in an activated state.

Transgenic plants (or plant cells, or plant explants, or plant tissues) incorporating the polynucleotides of the invention and/or expressing the polypeptides of the invention can be produced by a variety of well established techniques as described above. Following construction of a vector, most typically an expression cassette, including a polynucleotide, e.g., encoding a transcription factor or transcription factor homologue, described herein, standard techniques can be used to introduce the polynucleotide into a plant, a plant cell, a plant explant or a plant tissue of interest. Optionally, the plant cell, explant or tissue can be regenerated to produce a transgenic plant.

The plant can be any higher plant, including gymnosperms, monocotyledonous and dicotyledenous plants. Suitable protocols are available for *Leguminosae* (alfalfa, soybean, clover, etc.), *Umbelliferae* (carrot, celery, parsnip), *Cruciferae* (cabbage, radish, rapeseed, broccoli, etc.), *Curcurbitaceae* (melons and cucumber), *Gramineae* (wheat, com, rice, barley, millet, etc.), *Solanaceae* (potato, tomato, tobacco, peppers, etc.), and various other crops. See protocols. described in Ammirato et al. (1984) Handbook of Plant Cell Culture -Crop Species. Macmillan Publ. Co. Shimamoto et al. (1989) Nature 338:274-276; Fromm et al. (1990) Bio/Technology 8:833-839; and Vasil et al. (1990) Bio/Technology 8:429-434.

Transformation and regeneration of both monocotyledonous and dicotyledonous plant cells is now routine, and the selection of the most appropriate transformation technique will be determined by the practitioner. The choice of method will vary with the type of plant to be transformed; those skilled in the art will recognize the suitability of particular methods for given plant types. Suitable methods can include, but are not limited to: electroporation of plant protoplasts; liposome-mediated transformation; polyethylene glycol (PEG) mediated transformation; transformation using viruses; micro-injection of plant cells; micro-projectile bombardment of plant cells; vacuum infiltration; and *Agrobacterium tumeficiens* mediated transformation. Transformation means introducing a nucleotide sequence in a plant in a manner to cause stable or transient expression of the sequence.

Successful examples of the modification of plant characteristics by transformation with cloned sequences which serve to illustrate the current knowledge in this field of technology, includes: U.S. Patent Nos. 5,571,706; 5,677,175; 5.510,471; 5,750,386; 5,597,945; 5,589,615; 5,750,871; 5,268,526; 5,780,708; 5,538,880; 5,773,269; 5,736,369 and 5,610,042.

Following transformation, plants are preferably selected using a dominant selectable marker incorporated into the transformation vector. Typically, such a marker will confer antibiotic or herbicide resistance on the transformed plants, and selection of transformants can be accomplished by exposing the plants to appropriate concentrations of the antibiotic or herbicide.

After transformed plants are selected and grown to maturity, those plants showing a modified trait are identified. The modified trait can be any of those traits described above. Additionally, to confirm that the modifed trait is due to changes in expression levels or activity of the polypeptide or polynucleotide can be determined by analyzing mRNA expression using Northern blots, RT-PCR or microarrays, or protein expression using immunoblots or Western blots or gel shift assays.

### INTEGRATED SYSTEMS-SEQUENCE INDENT

Additionally, described herein is an integrated system, computer or computer readable medium that comprises an instruction set for determining the identity of one or more sequences in a database. In addition, the instruction set can be used to generate or identify sequences that meet any specified criteria. Furthermore, the instruction set may be used to associate or link certain functional benefits, such improved biochemical characteristics, with one or more identified sequence.

For example, the instruction set can include, e.g., a sequence comparison or other alignment program, e.g., an available program such as, for example, the Wisconsin Package Version 10.0, such as BLAST, FASTA, PILEUP, FINDPATTERNS or the like (GCG, Madision, WI). Public sequence databases such as GenBank, EMBL, Swiss-Prot and PIR or private sequence databases such as PhytoSeq (Incyte Pharmaceuticals, Palo Alto, CA) can be searched.

Alignment of sequences for comparison can be conducted by the local homology algorithm of Smith and Waterman (1981) Adv. Appl. Math. 2:482, by the homology alignment algorithm of Needleman and Wunsch (1970) J. Mol. Biol. 48:443, by the search for similarity method of Pearson and Lipman (1988) Proc. Natl. Acad. Sci. U.S.A. 85: 2444, by computerized implementations of these algorithms. After alignment, sequence comparisons between two (or more) polynucleotides or polypeptides are typically performed by comparing sequences of the two sequences over a comparison window to identify and compare local regions of sequence similarity. The comparison window can be a segment of at least about 20 contiguous positions, usually about 50 to about 200, more usually about 100 to about 150 contiguous positions. A description of the method is provided in Ausubel et al., *supra.*

A variety of methods of determining sequence relationships can be used, including manual alignment and computer assisted sequence alignment and analysis. This later approach is a preferred approach due to the increased throughput afforded by computer assisted methods. As noted above, a variety of computer programs for performing sequence alignment are available, or can be produced by one of skill.

One example algorithm that is suitable for determining percent sequence identity and sequence similarity is the BLAST algorithm, which is described in Altschul et al. J. Mol. Biol 215:403-410 (1990). Software for performing BLAST analyses is publicly available, e.g., through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., *supra).* These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (see Henikoff & Henikoff (1989) Proc. Natl. Acad. Sci. USA 89:10915).

In addition to calculating percent sequence identity, the BLAST algorithm also performs a statistical analysis of the similarity between two sequences (*see,* e.g., Karlin & Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence (and, therefore, in this context, homologous) if the smallest sum probability in a comparison of the test nucleic acid to the reference nucleic acid is less than about 0.1, or less than about 0.01, and or even less than about 0.001. An additional example of a useful sequence alignment algorithm is PILEUP. PILEUP creates a multiple sequence alignment from a group of related sequences using progressive, pairwise alignments. The program can align, e.g., up to 300 sequences of a maximum length of 5,000 letters.

The integrated system, or computer typically includes a user input interface allowing a user to selectively view one or more sequence records corresponding to the one or more character strings, as well as an instruction set which aligns the one or more character strings with each other or with an additional character string to identify one or more region of sequence similarity. The system may include a link of one or more character strings with a particular phenotype or gene function. Typically, the system includes a user readable output element which displays an alignment produced by the alignment instruction set.

The methods described herein can be implemented in a localized or distributed computing environment. In a distributed environment, the methods may implemented on a single computer comprising multiple processors or on a multiplicity of computers. The computers can be linked, e.g. through a common bus, but more preferably the computer(s) are nodes on a network. The network can be a generalized or a dedicated local or wide-area network and, in certain preferred embodiments, the computers may be components of an intra-net or an internet.

Thus, described herein are methods for identifying a sequence similar or homologous to one or more polynucleotides as noted herein, or one or more target polypeptides encoded by the polynucleotides, or otherwise noted herein and may include linking or associating a given plant phenotype or gene function with a sequence. In the methods, a sequence database is provided (locally or across an inter or intra net) and a query is made against the sequence database using the relevant sequences herein and associated plant phenotypes or gene functions.

Any sequence herein can be entered into the database, before or after querying the database. This provides for both expansion of the database and, if done before the querying step, for insertion of control sequences into the database. The control sequences can be detected by the query to ensure the general integrity of both the database and the query. As noted, the query can be performed using a web browser based interface. For example, the database can be a centralized public database such as those noted herein, and the querying can be done from a remote terminal or computer across an internet or intranet.

### EXAMPLES

The following examples are intended to illustrate but not limit the present invention.

### EXAMPLE I. FULL LENGTH GENE IDENTIFICATION AND CLONING

Putative transcription factor sequences (genomic or ESTs) related to known transcription factors were identified in the *Arabidopsis thaliana* GenBank database using the tblastn sequence analysis program using default parameters and a P-value cutoff threshold of -4 or -5 or lower, depending on the length of the query sequence. Putative transcription factor sequence hits were then screened to identify those containing particular sequence strings. If the sequence hits contained such sequence strings, the sequences were confirmed as transcription factors.

Alternatively, Arabidopsis *thaliana* cDNA libraries derived from different tissues or treatments, or genomic libraries were screened to identify novel members of a transcription family using a low stringency hybridization approach. Probes were synthesized using gene specific primers in a standard PCR reaction (annealing temperature 60°C) and labeled with ³²P dCTP using the High Prime DNA Labeling Kit (Boehringer Mannheim). Purified radiolabelled probes were added to filters immersed in Church hybridization medium (0.5 M NaPO₄ pH 7.0, 7% SDS, 1 % w/v bovine serum albumin) and hybridized overnight at 60 °C with shaking. Filters were washed two times for 45 to 60 minutes with 1xSCC, 1% SDS at 60° C.

To identify additional sequence 5' or 3' of a partial cDNA sequence in a cDNA library, 5' and 3' rapid amplification of cDNA ends (RACE) was performed using the Marathon^{™} cDNA amplification kit (Clontech, Palo Alto, CA). Generally, the method entailed first isolating poly(A) mRNA, performing first and second strand cDNA synthesis to generate double stranded cDNA, blunting cDNA ends, followed by ligation of the Marathon^{™} Adaptor to the cDNA to form a library of adaptor-ligated ds cDNA.

Gene-specific primers were designed to be used along with adaptor specific primers for both 5' and 3' RACE reactions. Nested primers, rather than single primers, were used to increase PCR specificity. Using 5' and 3' RACE reactions, 5' and 3' RACE fragments were obtained, sequenced and cloned. The process can be repeated until 5' and 3' ends of the full-length gene were identified. Then the full-length cDNA was generated by PCR using primers specific to 5' and 3' ends of the gene by end-to-end PCR.

### EXAMPLE II. CONSTRUCTION OF EXPRESSION VECTORS

The sequence was amplified from a genomic or cDNA library using primers specific to sequences upstream and downstream of the coding region. The expression vector was pMEN20 or pMEN65, which are both derived from pMON316 (Sanders et al, (1987) Nucleic Acids Research 15:1543-58) and contain the CaMV 35S promoter to express transgenes. To clone the sequence into the vector, both pMEN20 and the amplified DNA fragment were digested separately with SalI and NotI restriction enzymes at 37°C for 2 hours. The digestion products were subject to electrophoresis in a 0.8% agarose gel and visualized by ethidium bromide staining. The DNA fragments containing the sequence and the linearized plasmid were excised and purified by using a Qiaquick gel extraction kit (Qiagen, CA). The fragments of interest were ligated at a ratio of 3:1 (vector to insert). Ligation reactions using T4 DNA ligase (New England Biolabs, MA) were carried out at 16° C for 16 hours. The ligated DNAs were transformed into competent cells of the E. constrain DH5alpha by using the heat shock method. The transformations were plated on LB plates containing 50 mg/l kanamycin (Sigma).

Individual colonies were grown overnight in five milliliters of LB broth containing 50 mg/l kanamycin at 37° C. Plasmid DNA was purified by using Qiaquick Mini Prep kits (Qiagen, CA).

### EXAMPLE III. TRANSFORMATION OF AGROBACTERIUM WITH THE EXPRESSION VECTOR

After the plasmid vector containing the gene was constructed, the vector was used to transform *Agrobacterium tumefaciens* cells expressing the gene products. The stock of *Agrobacterium tumefaciens* cells for transformation were made as described by Nagel et al. (1990) FEMS Microbiol Letts. 67: 325-328. *Agrobacterium* strain ABI was grown in 250 ml LB medium (Sigma) overnight at 28°C with shaking until an absorbance (A₆₀₀) of 0.5 - 1.0 was reached. Cells were harvested by centrifugation at 4,000 x g for 15 min at 4°C. Cells were then resuspended in 250 µl chilled buffer (1 mM HEPES, pH adjusted to 7.0 with KOH). Cells were centrifuged again as described above and resuspended in 125 µl chilled buffer. Cells were then centrifuged and resuspended two more times in the same HEPES buffer as described above at a volume of 100 µl and 750 µl, respectively. Resuspended cells were then distributed into 40 µl aliquots, quickly frozen in liquid nitrogen, and stored at -80° C.

*Agrobacterium* cells were transformed with plasmids prepared as described above following the protocol described by Nagel et al. For each DNA construct to be transformed, 50 - 100 ng DNA (generally resuspended in 10 mM Tris-HCl, 1 mM EDTA, pH 8.0) was mixed with 40 µl of *Agrobacterium* cells. The DNA/cell mixture was then transferred to a chilled cuvette with a 2mm electrode gap and subject to a 2.5 kV charge dissipated at 25 µF and 200 µF using a Gene Pulser II apparatus (Bio-Rad). After electroporation, cells were immediately resuspended in 1.0 ml LB and allowed to recover without antibiotic selection for 2 - 4 hours at 28° C in a shaking incubator. After recovery, cells were plated onto selective medium of LB broth containing 100 µg/ml spectinomycin (Sigma) and incubated for 24-48 hours at 28° C. Single colonies were then picked and inoculated in fresh medium. The presence of the plasmid construct was verified by PCR amplification and sequence analysis.

### EXAMPLE IV. TRANSFORMATION OF ARABIDOPSIS PLANTS WITH AGROBACTERIUM TUMEFACIENS WITH EXPRESSION VECTOR

After transformation of *Agrobacterium tumefaciens* with plasmid vectors containing the gene, single *Agrobacterium* colonies were identified, propagated, and used to transform *Arabidopsis* plants. Briefly, 500 ml cultures of LB medium containing 50 mg/l kanamycin were inoculated with the colonies and grown at 28° C with shaking for 2 days until an absorbance (A₆₀₀) of > 2.0 is reached. Cells were then harvested by centrifugation at 4,000 x g for 10 min, and resuspended in infiltration medium (1/2 X Murashige and Skoog salts (Sigma), 1 X Gamborg's B-5 vitamins (Sigma), 5.0% (w/v) sucrose (Sigma), 0.044 µM benzylamino purine (Sigma), 200 µl/L Silwet L-77 (Lehle Seeds) until an absorbance (A₆₀₀) of 0.8 was reached.

Prior to transformation, *Arabidopsis thaliana* seeds (ecotype Columbia) were sown at a density of ∼10 plants per 4" pot onto Pro-Mix BX potting medium (Hummert International) covered with fiberglass mesh (18 mm X 16 mm). Plants were grown under continuous illumination (50-75 µE/m²/sec) at 22-23° C with 65-70% relative humidity. After about 4 weeks, primary inflorescence stems (bolts) are cut off to encourage growth of multiple secondary bolts. After flowering of the mature secondary bolts, plants were prepared for transformation by removal of all siliques and opened flowers.

The pots were then immersed upside down in the mixture of *Agrobacterium* infiltration medium as described above for 30 sec, and placed on their sides to allow draining into a 1' x 2' flat surface covered with plastic wrap. After 24 h, the plastic wrap was removed and pots are turned upright. The immersion procedure was repeated one week later, for a total of two immersions per pot. Seeds were then collected from each transformation pot and analyzed following the protocol described below.

### EXAMPLE V. IDENTIFICATION OF ARABIDOPSIS PRIMARY TRANSFORMANTS

Seeds collected from the transformation pots were sterilized essentially as follows. Seeds were dispersed into in a solution containing 0.1% (v/v) Triton X-100 (Sigma) and sterile H₂O and washed by shaking the suspension for 20 min. The wash solution was then drained and replaced with fresh wash solution to wash the seeds for 20 min with shaking. After removal of the second wash solution, a solution containing 0.1% (v/v) Triton X-100 and 70% ethanol (Equistar) was added to the seeds and the suspension was shaken for 5 min. After removal of the ethanol/detergent solution, a solution containing 0.1% (v/v) Triton X-100 and 30% (v/v) bleach (Clorox) was added to the seeds, and the suspension was shaken for 10 min. After removal of the bleach/detergent solution, seeds were then washed five times in sterile distilled H₂O. The seeds were stored in the last wash water at 4°C for 2 days in the dark before being plated onto antibiotic selection medium (1 X Murashige and Skoog salts (pH adjusted to 5.7 with 1M KOH), 1 X Gamborg's B-5 vitamins, 0.9% phytagar (Life Technologies), and 50 mg/l kanamycin). Seeds were germinated under continuous illumination (50-75 µE/m²/sec) at 22-23° C. After 7-10 days of growth under these conditions, kanamycin resistant primary transformants (T₁ generation) were visible and obtained. These seedlings were transferred first to fresh selection plates where the seedlings continued to grow for 3-5 more days, and then to soil (Pro-Mix BX potting medium).

Primary transformants were crossed and progeny seeds (T₂) collected; kanamycin resistant seedlings were selected and analyzed. The expression levels of the recombinant polynucleotides in the transformants varies from about a 5% expression level increase to a least a 100% expression level increase. Similar observations are made with respect to polypeptide level expression.

### EXAMPLE VI. IDENTIFICATION OF ARABIDOPSIS PLANTS WITH TRANSCRIPTION FACTOR GENE KNOCKOUTS

The screening of insertion mutagenized *Arabidopsis* collections for null mutants in a known target gene was essentially as described in Krysan et al (1999) Plant Cell 11:2283-2290. Briefly, gene-specific primers, nested by 5-250 base pairs to each other, were designed from the 5' and 3' regions of a known target gene. Similarly, nested sets of primers were also created specific to each of the T-DNA or transposon ends (the "right" and "left" borders). All possible combinations of gene specific and T-DNA/transposon primers were used to detect by PCR an insertion event within or close to the target gene. The amplified DNA fragments were then sequenced which allows the precise determination of the T-DNA/transposon insertion point relative to the target gene. Insertion events within the coding or intervening sequence of the genes were deconvoluted from a pool comprising a plurality of insertion events to a single unique mutant plant for functional characterization. The method is described in more detail in Yu and Adam, US Application Serial No. 09/177,733 filed October 23, 1998.

### EXAMPLE VII. IDENTIFICATION OF MODIFIED BIOCHEMICAL CHARACTERISTICS PHENOTYPE IN OVEREXPRESSOR OR GENE KNOCKOUT PLANTS

Experiments were performed to identify those transformants or knockouts that exhibited modified biochemical characteristics. Among the biochemicals that were assayed were insoluble sugars, such as arabinose, fucose, galactose, mannose, rhamnose or xylose or the like; prenyl lipids, such as lutein, beta-carotene, xanthophyll-1, xanthophyll-2, chlorophylls A or B, or alpha-, delta- or gamma-tocopherol or the like; fatty acids, such as 16:0 (palmitic acid), 16:1 (palmitoleic acid), 18:0 (stearic acid), 18:1 (oleic acid), 18:2 (linoleic acid), 20:0, 18:3 (linolenic acid), 20:1 (eicosenoic acid), 20:2, 22:1 (erucic acid) or the like; waxes, such as by altering the levels of C29, C31, or C33 alkanes; sterols, such as brassicasterol, campesterol, stigmasterol, sitosterol or stigmastanol or the like, glucosinolates, protein or oil levels

Fatty acids were measured using two methods depending on whether the tissue was from leaves or seeds. For leaves, lipids were extracted and esterified with hot methanolic H2SO4 and partitioned into hexane from methanolic brine. For seed fatty acids, seeds were pulverized and extracted in methanol:heptane:toluene:2,2-dimethoxypropane:H2SO4 (39:34:20:5:2) for 90 minutes at 80°C. After cooling to room temperature the upper phase, containing the seed fatty acid esters, was subjected to GC analysis. Fatty acid esters from both seed and leaf tissues were analyzed with a Supelco SP-2330 column.

Glucosinolates were purified from seeds or leaves by first heating the tissue at 95°C for 10 minutes. Preheated ethanol:water (50:50) is and after heating at 95°C for a further 10 minutes, the extraction solvent is applied to a DEAE Sephadex column which had been previously equilibrated with 0.5 M pyridine acetate. Desulfoglucosinolates were eluted with 300 ul water and analyzed by reverse phase HPLC monitoring at 226 nm.

For wax alkanes, samples were extracted using an identical method as fatty acids and extracts were analyzed on a HP 5890 GC coupled with a 5973 MSD. Samples were chromatographed on a J&W DB35 mass spectrometer (J&W Scientific).

To measure prenyl lipids levels, seeds or leaves were pulverized with 1 to 2% pyrogallol as an antioxidant. For seeds, extracted samples were filtered and a portion removed for tocopherol and carotenoid/chlorophyll analysis by HPLC. The remaining material was saponified for sterol determination. For leaves, an aliquot was removed and diluted with methanol and chlorophyll A, chlorophyll B, and total carotenoids measured by spectrophotometry by determining absorbance at 665.2 nm, 652.5 nm, and 470 nm. An aliquot was removed for tocopherol and carotenoid/chlorophyll composition by HPLC using a Waters uBondapak C18 column (4.6 mm x 150 mm). The remaining methanolic solution was saponified with 10% KOH at 80°C for one hour. The samples were cooled and diluted with a mixture of methanol and water. A solution of 2% methylene chloride in hexane was mixed in and the samples were centrifuged. The aqueous methanol phase was again re-extracted 2% methylene chloride in hexane and, after centrifugation, the two upper phases were combined and evaporated. 2% methylene chloride in hexane was added to the tubes and the samples were then extracted with one ml of water. The upper phase was removed, dried, and resuspended in 400 ul of 2% methylene chloride in hexane and analyzed by gas chromatography using a 50 m DB-5ms (0.25 mm ID, 0.25 um phase, J&W Scientific).

Insoluble sugar levels were measured by the method essentially described by Reiter et al., Plant Journal 12:335-345. This method analyzes the neutral sugar composition of cell wall polymers found in *Arabidopsis* leaves. Soluble sugars were separated from sugar polymers by extracting leaves with hot 70% ethanol. The remaining residue containing the insoluble polysaccharides was then acid hydrolyzed with allose added as an internal standard. Sugar monomers generated by the hydrolysis were then reduced to the corresponding alditols by treatment with NaBH4, then were acetylated to generate the volatile alditol acetates which were then analyzed by GC-FID. Identity of the peaks was determined by comparing the retention times of known sugars converted to the corresponding alditol acetates with the retention times of peaks from wild-type plant extracts. Alditol acetates were analyzed on a Supelco SP-2330 capillary column (30 m x 250 um x 0.2 um) using a temperature program beginning at 180° C for 2 minutes followed by an increase to 220° C in 4 minutes. After holding at 220°C for 10 minutes, the oven temperature is increased to 240° C in 2 minutes and held at this temperature for 10 minutes and brought back to room temperature.

To identify plants with alterations in total seed oil or protein content, 150mg of seeds from T2 progeny plants were subjected to analysis by Near Infrared Reflectance (NIR) using a Foss NirSystems Model 6500 with a spinning cup transport system.

Table 3 shows the phenotypes observed for particular overexpressor or knockout plants and provides the SEQ ID No., the internal reference code (GID), whether a knockout or overexpressor plant was analyzed and the observed phenotype.

**Table 3**

| SEQ ID No. | GID | Knockout (KO) or overexpressor (OE) | Phenotype observed |
|---|---|---|---|
| | G975 | OE | Up to 13-fold increase in wax in leaves |

For a particular overexpressor that shows a less beneficial biochemical characteristic, it may be more useful to select a plant with a decreased expression of the particular transcription factor. For a particular knockout that shows a less beneficial biochemical characteristic, it may be more useful to select a plant with an increased expression of the particular transcription factor.

### EXAMPLE VIII. IDENTIFICATION OF HOMOLOGOUS SEQUENCES

Homologous sequences from *Arabidopsis* and plant species other than *Arabidopsis* were identified using database sequence search tools, such as the Basic Local Alignment Search Tool (BLAST) (Altschul et al. (1990) J. Mol. Biol. 215:403-410; and Altschul et al. (1997) Nucl. Acid Res. 25: 3389-3402). The tblastx sequence analysis programs were employed using the BLOSUM-62 scoring matrix (Henikoff, S. and Henikoff, J. G. (1992) Proc. Natl. Acad. Sci. USA 89: 10915-10919). G1337 (SEQ ID NO:43) was identified as an *Arabidopsis* homologous sequence Additionally, the entire NCBI GenBank database was filtered for sequences from all plants except *Arabidopsis thaliana* by selecting all entries in the NCBI GenBank database associated with NCBI taxonomic ID 33090 (Viridiplantae; all plants) and excluding entries associated with taxonomic ID 3701 (*Arabidopsis thaiiana*). These sequences were compared to sequences representing genes of SEQ IDs Nos. 1-54 on 9/26/2000 using the Washington University TBLASTX algorithm (version 2.0a 19MP). For each gene of SEQ IDs Nos. 1-54, individual comparisons were ordered by probability score (P-value), where the score reflects the probability that a particular alignment occurred by chance. For example, a score of 3.6e-40 is 3.6 x 10⁻⁴⁰. For up to ten species, the gene with the lowest P-value (and therefore the most likely homolog) is listed in Figure 1.

In addition to P-values, comparisons were also scored by percentage identity. Percentage identity reflects the degree to which two segments of DNA or protein are identical over a particular length. The ranges of percent identity between the non-Arabidopsis genes shown in Figure 3 and the Arabidopsis genes in the sequence listing are: SEQ ID No. 43: 36%-70%.

The polynucleotides and polypeptides in the Sequence Listing and the identified homologous sequences may be stored in a computer system and have associated or linked with the sequences a function, such as that the polynucleotides and polypeptides are useful for modifying the biochemical characteristics of a plant.

## Claims

1. Use of a nucleotide sequence which encodes a transcription factor polypeptide comprising a sequence having at least 50% sequence identity to SEQ ID NO:28 for producing a transgenic plant having the trait of increased wax content in leaves by over-expression of said polypeptide.

2. Use of claim 1 wherein the transcription factor polypeptide comprises a sequence having at least 80% sequence identity to SEQ ID NO:28.

3. Use of claim 1 wherein said polypeptide comprises SEQ 10 NO:28.

4. Use of any one of claims 1 to 3 wherein said increase in wax content in leaves is at least a 100% increase in wax content in leaves.

5. Use of any one of the preceding claims wherein said nucleotide sequence which encodes a polypeptide is operably linked to a constitutive, inducible or tissue-active promoter.

6. Use of any one of the preceding claims wherein said plant is soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, and vegetable brassicas.

7. A method for producing transgenic plant having the trait of increased wax content in leaves, said method comprising:
introducing into a plant, plant cell, plant explant or plant tissue an expression vector comprising a nucleotide sequence which encodes a transcription factor polypeptide comprising a sequence having at least 50% sequence identity to the SEQ ID NO:28;
producing a transgenic plant having said expression vector encoding said transcription factor polypeptide; and
selecting a plant having said trait of increased wax content in leaves caused by over-expression of said polypeptide.

8. The method of claim 7 wherein the transcription factor polypeptide comprises a sequence having at least 80% sequence identity to the SEQ ID NO:28.

9. The method of claim 7 wherein said vector comprises a nucleotide sequence which encodes the polypeptide of SEQ ID NO:28.

10. The method of any one of claims 7. 8 or 9 wherein said selecting is selecting for the trait of at least a 100% increase in said wax in leaves.

11. The method of any one of claims 7 to 10 wherein said nucleotide sequence which encodes a polypeptide is operably linked to a constitutive, inducible or tissue-active promoter.

12. The method of any one of claims 7 to 11 wherein said plant is soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, and vegetable brassicas.

13. A transgenic plant produced by the method of any one of claims 7 to 10, or progeny thereof, wherein said plant or progeny thereof comprises an expression vector comprising a nucleotide sequence which encodes a polypeptide having at least 50% sequence identity to SEQ ID NO:28 and said plant has the trait of increased wax content in leaves caused by ectopic over-expression of said transcription factor polypeptide.

14. The transgenic plant of claim 13 wherein the transcription factor polypeptide comprises a sequence having at least 80% sequence identity to SEQ ID NO:28.

15. The transgenic plant of claim 13 or 14, wherein said polypeptide comprises SEQ ID NO:28.

16. The transgenic plant of claim 13, 14 or 15 wherein said plant is soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, and vegetable brassicas.

17. Seed of the transgenic plant of any one of claims 13 to 16, said seed comprising an expression vector comprising a nucleotide sequence which encodes a polypeptide having at least 50% sequence identity to SEQ ID NO:28.

18. Seed of claim 17 wherein the transcription factor polypeptide comprises a sequence having at least 80% sequence identity to SEQ ID NO:28.

## Patentansprüche

1. Verwendung einer Nucleotidsequenz, die für ein Transkriptionsfaktor-Polypeptid kodiert, das eine Sequenz mit zumindest 50 % Sequenzidentität mit Seq.-ID Nr. 28 umfasst, zur Herstellung einer transgenen Pflanze mit dem Merkmal erhöhten Wachsgehalts in Blättern durch Überexpression des Polypeptids.

2. Verwendung nach Anspruch 1, wobei das Transkriptionsfaktor-Polypeptid eine Sequenz mit zumindest 80 % Sequenzidentität mit Seq.-ID Nr. 28 umfasst.

3. Verwendung nach Anspruch 1, wobei das Polypeptid Seq.-ID Nr. 28 umfasst.

4. Verwendung nach einem der Ansprüche 1 bis 3, wobei die Erhöhung des Wachsgehalts in Blättern eine zumindest 100%ige Erhöhung des Wachsgehalts in Blättern ist.

5. Verwendung nach einem der vorangegangenen Ansprüche, wobei die Nucleotidsequenz, die für ein Polypeptid kodiert, operabel an einen konstitutiven, induzierbaren oder gewebeaktiven Promotor gebunden ist.

6. Verwendung nach einem der vorangegangenen Ansprüche, wobei die Pflanze Soja, Weizen, Mais, Kartoffel, Baumwolle, Reis, Raps, Sonnenblume, Luzerne, Zuckerrohr, Rasen, Banane, Brombeere, Heidelbeere, Erdbeere, Himbeere, Kantalupe, Karotte, Blumenkohl, Kaffee, Gurke, Aubergine, Traube, Honigmelone, Kopfsalat, Mango, Melone, Zwiebel, Papaya, Erbse, Paprika, Ananas, Spinat, Kürbis, Zuckermais, Tabak, Tomate, Wassermelone, Früchte von Rosengewächsen oder Gemüsekohl ist.

7. Verfahren zur Herstellung einer transgenen Pflanze mit dem Merkmal eines erhöhten Wachsgehalts in Blättern, wobei das Verfahren Folgendes umfasst:
Einführen eines Expressionsvektors, der eine Nucleotidsequenz umfasst, die für ein Transkriptionsfaktor-Polypeptid kodiert, das eine Sequenz mit zumindest 50 % Sequenzidentität mit Seq.-ID Nr. 28 aufweist, in eine Pflanze, eine Pflanzenzelle, ein Pflanzenexplantat oder Pflanzengewebe;
Produzieren einer transgenen Pflanze mit dem Expressionsvektor, der für das Transkriptionsfaktor-Polypeptid kodiert; und
Auswählen einer Pflanze mit dem Merkmal eines erhöhten Wachsgehalts in Blättern, der durch die Überexpression des Polypeptids verursacht wird.

8. Verfahren nach Anspruch 7, wobei das Transkriptionsfaktor-Polypeptid eine Sequenz mit zumindest 80 % Sequenzidentität mit Seq.-ID Nr. 28 umfasst.

9. Verfahren nach Anspruch 7, wobei der Vektor eine Nucleotidsequenz umfasst, die für das Polypeptid der Seq.-ID Nr. 28 kodiert.

10. Verfahren nach einem der Ansprüche 7, 8 oder 9, wobei das Auswählen das Auswählen bezüglich des Merkmals einer zumindest 100%igen Steigerung des Wachses in Blättern ist.

11. Verfahren nach einem der Ansprüche 7 bis 10, wobei die Nucleotidsequenz, die für ein Polypeptid kodiert, operabel an einen konstitutiven, induzierbaren oder gewebeaktiven Promotor gebunden ist.

12. Verfahren nach einem der Ansprüche 7 bis 11, wobei die Pflanze Soja, Weizen, Mais, Kartoffel, Baumwolle, Reis, Raps, Sonnenblume, Luzerne, Zuckerrohr, Rasen, Banane, Brombeere, Heidelbeere, Erdbeere, Himbeere, Kantalupe, Karotte, Blumenkohl, Kaffee, Gurke, Aubergine, Traube, Honigmelone, Kopfsalat, Mango, Melone, Zwiebel, Papaya, Erbse, Paprika, Ananas, Spinat, Kürbis, Zuckermais, Tabak, Tomate, Wassermelone, Früchte von Rosengewächsen oder Gemüsekohl ist.

13. Transgene Pflanze, die durch ein Verfahren nach einem der Ansprüche 7 bis 10 hergestellt ist, oder Nachkommenschaft davon, wobei die Pflanze oder die Nachkommenschaft davon einen Expressionsvektor umfasst, der eine Nucleotidsequenz umfasst, die für ein Polypeptid mit zumindest 50 % Sequenzidentität mit Seq.-ID Nr. 28 kodiert, und die Pflanze das Merkmal eines erhöhten Wachsgehalts in Blättern aufweist, der durch ektopische Überexpression des Transkriptionsfaktor-Polypeptids verursacht wird.

14. Transgene Pflanze nach Anspruch 13, wobei das Transkriptionsfaktor-Polypeptid eine Sequenz mit zumindest 80 % Sequenzidentität mit Seq.-ID Nr. 28 umfasst.

15. Transgene Pflanze nach Anspruch 13 oder 14, wobei das Polypeptid Seq.-ID Nr. 28 umfasst.

16. Transgene Pflanze nach Anspruch 13, 14 oder 15, wobei die Pflanze Soja, Weizen, Mais, Kartoffel, Baumwolle, Reis, Raps, Sonnenblume, Luzerne, Zuckerrohr, Rasen, Banane, Brombeere, Heidelbeere, Erdbeere, Himbeere, Kantalupe, Karotte, Blumenkohl, Kaffee, Gurke, Aubergine, Traube, Honigmelone, Kopfsalat, Mango, Melone, Zwiebel, Papaya, Erbse, Paprika, Ananas, Spinat, Kürbis, Zuckermais, Tabak, Tomate, Wassermelone, Früchte von Rosengewächsen oder Gemüsekohl ist.

17. Samen einer transgenen Pflanze nach einem der Ansprüche 13 bis 16, wobei der Samen einen Expressionsvektor umfasst, der eine Nucleotidsequenz umfasst, die für ein Polypeptid mit zumindest 50 % Sequenzidentität mit Seq.-ID Nr. 28 kodiert.

18. Samen nach Anspruch 17, wobei das Transkriptionsfaktor-Polypeptid eine Sequenz mit zumindest 80 % Sequenzidentität mit Seq.-ID Nr. 28 umfasst.

## Revendications

1. Utilisation d'une séquence de nucléotides qui code pour un polypeptide à facteur de transcription comprenant une séquence ayant au moins 50% d'identité de séquence avec la SEQ ID NO:28 pour produire une plante transgénique ayant le trait d'une teneur en cire augmentée dans les feuilles par la surexpression dudit polypeptide.

2. Utilisation selon la revendication 1, où le polypeptide à facteur de transcription comprend une séquence ayant au moins 80% d'identité de séquence avec la SEQ ID NO:28.

3. Utilisation selon la revendication 1, où ledit polypeptide comprend la SEQ ID NO:28.

4. Utilisation selon l'une quelconque des revendications 1 à 3, où ladite augmentation dans la teneur en cire dans les feuilles représente au moins une augmentation de 100% dans la teneur en cire dans les feuilles.

5. Utilisation selon l'une quelconque des revendications précédentes, où ladite séquence de nucléotides qui code pour un polypeptide est fonctionnellement liée à un promoteur constitutif, inductible ou actif pour le tissu.

6. Utilisation selon l'une quelconque des revendications précédentes, où ladite plante est le soja, blé, maïs, pomme de terre, coton, riz, navette de graines oléagineuses, tournesol, luzerne, canne à sucre, tourbe, banane, cassis, myrtille, fraise, framboise, melon brodé, carotte, chou-fleur, café, concombre, aubergine, raisin, miellat, laitue, mangue, melon, oignon, papaye, petits pois, poivres, ananas, épinards, courge, maïs sucré, tabac, tomate, melon doux, fruits rosacés et crucifères végétaux.

7. Méthode de production d'une plante transgénique ayant le trait d'une teneur en cire augmentée dans les feuilles, ladite méthode comprenant:
introduire dans une plante, cellule de plante, explant de plante ou tissu de plante un vecteur d'expression comprenant une séquence de nucléotides qui code pour un polypeptide à facteur de transcription comprenant une séquence ayant au moins 50% d'identité de séquence avec la SEQ ID NO:28;
produire une plante transgénique ayant ledit vecteur d'expression codant pour ledit polypeptide à facteur de transcription; et
sélectionner une plante ayant ledit trait d'une teneur en cire augmentée dans les feuilles provoquée par la surexpression dudit polypeptide.

8. Méthode selon la revendication 7, où le polypeptide à facteur de transcription comprend une séquence ayant au moins 80% d'identité de séquence avec la SEQ ID NO:28.

9. Méthode selon la revendication 7, où ledit vecteur comprend une séquence de nucléotides qui code pour le polypeptide de la SEQ ID NO:28.

10. Méthode selon l'une quelconque des revendications 7, 8 ou 9, où ladite sélection est la sélection pour le trait d'au moins 100% d'augmentation dans ladite cire dans les feuilles.

11. Méthode selon l'une quelconque des revendications 7 à 10, où ladite séquence de nucléotides qui code pour un polypeptide est fonctionnellement liée à un promoteur constitutif, inductible ou actif pour le tissu.

12. Méthode selon l'une quelconque des revendications 7 à 11, où ladite plante est le soja, blé, maïs, pomme de terre, coton, riz, navette de graines oléagineuses, tournesol, luzerne, canne à sucre, tourbe, banane, cassis, myrtille, fraise, framboise, melon brodé, carotte, chou-fleur, café, concombre, aubergine, raisin, miellat, laitue, mangue, melon, oignon, papaye, petits pois, poivres, ananas, épinards, courge, maïs sucré, tabac, tomate, melon doux, fruits rosacés et crucifères végétaux.

13. Plante transgénique produite par la méthode selon l'une quelconque des revendications 7 à 10 ou une progéniture de celle-ci, où ladite plante ou progéniture de celle-ci comprend un vecteur d'expression comprenant une séquence de nucléotides qui code pour un polypeptide ayant au moins 50% d'identité de séquence avec la SEQ ID NO:28, et ladite plante a le trait d'une teneur en cire augmentée dans les feuilles provoquée par la surexpression ectopique dudit polypeptide à facteur de transcription.

14. Plante transgénique selon la revendication 13, où le polypeptide à facteur de transcription comprend une séquence ayant au moins 80% d'identité de séquence avec la SEQ ID NO:28.

15. Plante transgénique selon la revendication 13 ou 14, où ledit polypeptide comprend la SEQ ID NO:28.

16. Plante transgénique selon la revendication 13, 14 ou 15, où ladite plante est le soja, blé, maïs, pomme de terre, coton, riz, navette de graines oléagineuses, tournesol, luzerne, canne à sucre, tourbe, banane, cassis, myrtille, fraise, framboise, melon brodé, carotte, chou-fleur, café, concombre, aubergine, raisin, miellat, laitue, mangue, melon, oignon, papaye, petits pois, poivres, ananas, épinards, courge, maïs sucré, tabac, tomate, melon doux, fruits rosacés et crucifères végétaux.

17. Semence de la plante transgénique selon l'une quelconque des revendications 13 à 16, ladite semence comprend un vecteur d'expression comprenant une séquence de nucléotides qui code pour un polypeptide ayant au moins 50% d'identité de séquence avec la SEQ ID NO:28.

18. Semence selon la revendication 17, où le polypeptide à facteur de transcription comprend une séquence ayant au moins 80% d'identité de séquence avec la SEQ ID NO:28.


### SEQUENCE LISTING

<110> Mendel Biotechnology, Inc.
<120> Plant Biochemistry-Related Genes
<130> 6463111
<140> EP00980390.9
   <141> 2000-11-14
<150> 60/164,132
<151> 1999-11-17
<150> 60/197,899
<151> 2000-04-17
<150> 60/227,439
<151> 2000-08-22
<160> 44
<170> PatentIn version 3.0
<210> 1
<400> 1
   000
<210> 2
<400> 2
   000
<210> 3
<400> 3
   000
<210> 4
<400> 4
   000
<210> 5
<400> 5
000
<210> 6
<400> 6
   000
<210> 7
<400> 7
   000
<210> 8
<400> 8
   000
<210> 9
<400> 9
   000
<210> 10
<400> 10
   000
<210> 11
<400> 11
   000
<210> 12
<400> 12
   000
<210> 13
<400> 13
   000
<210> 14
<400> 14
   000
<210> 15
<400> 15
   000
<210> 16
<400> 16
   000
<210> 17
<400> 17
   000
<210> 18
<400> 18
   000
<210> 19
<400> 19
   000
<210> 20
<400> 20
   000
<210> 21
<400> 21
   000
<210> 22
<400> 22
   000
<210> 23
<400> 23
   000
<210> 24
<400> 24
   000
<210> 25
<400> 25
   000
<210> 26
<400> 26
   000
<210> 27
<211> 768
<212> DNA
<213> Arabidopsis thaliana
<220>
<221> CDS
<222> (58)..(657)
<223> G975
<400> 27
   attactcatc atcaagttcc tactttctct ctgacaaaca tcacagagta agtaaga 57
   atg gta cag acg aag aag ttc aga ggt gtc agg caa cgc cat tgg ggt 105
   Met Val Gln Thr Lys Lys Phe Arg Gly Val Arg Gln Arg His Trp Gly
   1               5                   10                  15
   tct tgg gtc gct gag att cgt cat cct ctc ttg aaa cgg agg att tgg      153
   Ser Trp Val Ala Glu Ile Arg His Pro Leu Leu Lys Arg Arg Ile Trp
               20                  25                  30
   cta ggg acg ttc gag acc gca gag gag gca gca aga gca tac gac gag      201
   Leu Gly Thr Phe Glu Thr Ala Glu Glu Ala Ala Arg Ala Tyr Asp Glu
           35                  40                  45
   gcc gcc gtt tta atg agc ggc cgc aac gcc aaa acc aac ttt ccc ctc      249
   Ala Ala Val Leu Met Ser Gly Arg Asn Ala Lys Thr Asn Phe Pro Leu
       50                  55                  60
   aac aac aac aac acc gga gaa act tcc gag ggc aaa acc gat att tca      297
   Asn Asn Asn Asn Thr Gly Glu Thr Ser Glu Gly Lys Thr Asp Ile Ser
   65                  70                  75                  80
   gct tcg tcc aca atg tca tcc tca aca tca tct tca tcg ctc tct tcc      345
   Ala Ser Ser Thr Met Ser Ser Ser Thr Ser Ser Ser Ser Leu Ser Ser
                   85                  90                  95
   atc ctc agc gcc aaa ctg agg aaa tgc tgc aag tct cct tcc cca tcc      393
   Ile Leu Ser Ala Lys Leu Arg Lys Cys Cys Lys Ser Pro Ser Pro Ser
               100                 105                 110
   ctc acc tgc ctc cgt ctt gac aca gcc agc tcc cat atc ggc gtc tgg      441
   Leu Thr Cys Leu Arg Leu Asp Thr Ala Ser Ser His Ile Gly Val Trp
           115                 120                 125
   cag aaa cgg gcc ggt tca aag tct gac tcc agc tgg gtc atg acg gtg      489
   Gln Lys Arg Ala Gly Ser Lys Ser Asp Ser Ser Trp Val Met Thr Val
       130                 135                 140
   gag cta ggt ccc gca agc tcc tcc caa gag act act agt aaa gct tca      537
   Glu Leu Gly Pro Ala Ser Ser Ser Gln Glu Thr Thr Ser Lys Ala Ser
   145                 150                 155                 160
   caa gac gct att ctt gct ccg acc act gaa gtt gaa att ggt ggc agc      585
   Gln Asp Ala Ile Leu Ala Pro Thr Thr Glu Val Glu Ile Gly Gly Ser
                   165                 170                 175
   aga gaa gaa gta ttg gat gag gaa gaa aag gtt gct ttg caa atg ata      633
   Arg Glu Glu Val Leu Asp Glu Glu Glu Lys Val Ala Leu Gln Met Ile
               180                 185                 190
   gag gag ctt ctc aat aca aac taa atcttatttg cttatatata tgtacctatt     687
   Glu Glu Leu Leu Asn Thr Asn
           195
   ttcattgctg atttacagcc aaaataatca attataccgt gtattttata gatgttttat    747
   attaaaaggt tgttagatat a                                              768
<210> 28
<211> 199
<212> PRT
<213> Arabidopsis thaliana
<400> 28
   Met Val Gln Thr Lys Lys Phe Arg Gly Val Arg Gln Arg His Trp Gly
   1 5 10 15
   Ser Trp Val Ala Glu Ile Arg His Pro Leu Leu Lys Arg Arg Ile Trp
   20 25 30
   Leu Gly Thr Phe Glu Thr Ala Glu Glu Ala Ala Arg Ala Tyr Asp Glu
   35 40 45
   Ala Ala Val Leu Met Ser Gly Arg Asn Ala Lys Thr Asn Phe Pro Leu
   50 55 60
   Asn Asn Asn Asn Thr Gly Glu Thr Ser Glu Gly Lys Thr Asp Ile Ser
   65 70 75 80
   Ala Ser Ser Thr Met Ser Ser Ser Thr Ser Ser Ser Ser Leu Ser Ser
   85 90 95
   Ile Leu Ser Ala Lys Leu Arg Lys Cys Cys Lys Ser Pro Ser Pro Ser
   100 105 110
   Leu Thr Cys Leu Arg Leu Asp Thr Ala Ser Ser His Ile Gly Val Trp
   115 120 125
   Gln Lys Arg Ala Gly Ser Lys Ser Asp Ser Ser Trp Val Met Thr Val
   130 135 140
   Glu Leu Gly Pro Ala Ser Ser Ser Gln Glu Thr Thr Ser Lys Ala Ser
   145 150 155 160
   Gln Asp Ala Ile Leu Ala Pro Thr Thr Glu Val Glu Ile Gly Gly Ser
   165 170 175
   Arg Glu Glu Val Leu Asp Glu Glu Glu Lys Val Ala Leu Gln Met Ile
   180 185 190
   Glu Glu Leu Leu Asn Thr Asn
   195
<210> 29
<400> 29
   000
<210> 30
<400> 30
   000
<210> 31
<400> 31
   000
<210> 32
<400> 32
   000
<210> 33
<400> 33
   000
<210> 34
<400> 34
   000
<210> 35
<400> 35
   000
<210> 36
<400> 36
   000
<210> 37
<400> 37
   000
<210> 38
<400> 38
   000
<210> 39
<400> 39
   000
<210> 40
<400> 40
   000
<210> 41
<400> 41
   000
<210> 42
<400> 42
   000
<210> 43
<211> 844
<212> DNA
<213> Arabidopsis thaliana
<220>
<221> CDS <222> (89)..(658)
<223> G1387
<400> 43
   tctctctccc actctcactt tctctcctat tcttagttcg tgtcagaaac acacagagaa      60
   attaagaacc ctaatttaaa acagaaga atg gta cat tcg aag aag ttc cga        112
                                  Met Val His Ser Lys Lys Phe Arg
                                  1               5
   ggt gtc cgc cag cgt cag tgg ggt tct tgg gtt tct gag att cgt cat       160
   Gly Val Arg Gln Arg Gln Trp Gly Ser Trp Val Ser Glu Ile Arg His
       10                  15                  20
   cct ctc ttg aag aga aga gtg tgg cta gga aca ttc gac acg gcg gaa       208
   Pro Leu Leu Lys Arg Arg Val Trp Leu Gly Thr Phe Asp Thr Ala Glu
   25                  30                  35                  40
   aca gcg gct aga gcc tac gac caa gcc gcg gtt cta atg aac ggc cag       256
   Thr Ala Ala Arg Ala Tyr Asp Gln Ala Ala Val Leu Met Asn Gly Gln
                   45                  50                  55
   agc gcg aag act aac ttc ccc gtc atc aaa tcg aac ggt tca aat tcc       304
   Ser Ala Lys Thr Asn Phe Pro Val Ile Lys Ser Asn Gly Ser Asn Ser
               60                  65                  70
   ttg gag att aac tct gcg tta agg tct ccc aaa tca tta tcg gaa cta       352
   Leu Glu Ile Asn Ser Ala Leu Arg Ser Pro Lys Ser Leu Ser Glu Leu
           75                  80                  85
   ttg aac gct aag cta agg aag aac tgt aaa gac cag aca ccg tat ctg       400
   Leu Asn Ala Lys Leu Arg Lys Asn Cys Lys Asp Gln Thr Pro Tyr Leu
       90                  95                  100
   acg tgt ctc cgc ctc gac aac gac agc tca cac atc ggc gtc tgg cag       448
   Thr Cys Leu Arg Leu Asp Asn Asp Ser Ser His Ile Gly Val Trp Gln
   105                 110                 115                 120
   aaa cgc gcc ggg tca aaa acg agt cca aac tgg gtc aag ctt gtt gaa       496
   Lys Arg Ala Gly Ser Lys Thr Ser Pro Asn Trp Val Lys Leu Val Glu
                   125                 130                 135
   cta ggt gac aaa gtt aac gca cgt ccc ggt ggt gat att gag act aat       544
   Leu Gly Asp Lys Val Asn Ala Arg Pro Gly Gly Asp Ile Glu Thr Asn
               140                 145                 150
   aag atg aag gta cga aac gaa gac gtt cag gaa gat gat caa atg gcg       592
   Lys Met Lys Val Arg Asn Glu Asp Val Gln Glu Asp Asp Gln Met Ala
           155                 160                 165
   atg cag atg atc gag gag ttg ctt aac tgg acc tgt cct gga tct gga       640
   Met Gln Met Ile Glu Glu Leu Leu Asn Trp Thr Cys Pro Gly Ser Gly
       170                 175                 180
   tcc att gca cag gtc taa aggagaatca ttgaattata tgatcaagat              688
   Ser Ile Ala Gln Val
   185
   aataatatag ttgagggtta ataataatcg agggtaagta atttacgtgt agctaataat      748
   taatataatt ttcgaacata tatatgaata tatgatagct ctagaaatga gtacgtatat 808
   atacgtaaac atttttcctc aaatatagta tatgtg 844
<210> 44
<211> 189
<212> PRT
<213> Arabidopsis thaliana
<400> 44
   Met Val His Ser Lys Lys Phe Arg Gly Val Arg Gln Arg Gln Trp Gly
   1               5                   10                  15
   Ser Trp Val Ser Glu Ile Arg His Pro Leu Leu Lys Arg Arg Val Trp
               20                  25                  30
   Leu Gly Thr Phe Asp Thr Ala Glu Thr Ala Ala Arg Ala Tyr Asp Gln
           35                  40                  45
   Ala Ala Val Leu Met Asn Gly Gln Ser Ala Lys Thr Asn Phe Pro Val
       50                  55                  60
   Ile Lys Ser Asn Gly Ser Asn Ser Leu Glu Ile Asn Ser Ala Leu Arg
   65                  70                  75                  80
   Ser Pro Lys Ser Leu Ser Glu Leu Leu Asn Ala Lys Leu Arg Lys Asn
                   85                  90                  95
   Cys Lys Asp Gln Thr Pro Tyr Leu Thr Cys Leu Arg Leu Asp Asn Asp
               100                 105                 110
   Ser Ser His Ile Gly Val Trp Gln Lys Arg Ala Gly Ser Lys Thr Ser
           115                 120                 125
   Pro Asn Trp Val Lys Leu Val Glu Leu Gly Asp Lys Val Asn Ala Arg
       130                 135                 140
   Pro Gly Gly Asp Ile Glu Thr Asn Lys Met Lys Val Arg Asn Glu Asp
   145                 150                 155                 160
   Val Gln Glu Asp Asp Gln Met Ala Met Gln Met Ile Glu Glu Leu Leu
                   165                 170                 175
   Asn Trp Thr Cys Pro Gly Ser Gly Ser Ile Ala Gln Val
               180                 185